# EUROPEAN PATENT APPLICATION

(11) **EP 4 253 374 A1**
(43) Date of publication of application: **04.10.2023**
(21) Application number: 21897208.1
(22) Date of filing: 29.11.2021
(51) Int. Cl.: C07D 401/14, C07D 417/04, A61K 31/501, A61P 35/00

(54) **DEUTERATED 2-ARYLHETEROCYCLE-3-OXO-2,3-DIHYDROPYRIDAZINE-4-CARBOXAMIDE INHIBITOR AND PREPARATION METHOD THEREFOR AND APPLICATION THEREOF**

(30) Priority: 27.11.2020 CN 202011367513
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Shanghai 201203 (CN); CUI, Dawei, Shanghai 201203 (CN); LIU, Lianjun, Shanghai 201203 (CN); PANG, Xudong, Shanghai 201203 (CN); CHAI, Chuanke, Shanghai 201203 (CN)
(74) Representative: dompatent von Kreisler Selting Werner - Partnerschaft von Patent- und Rechtsanwälten mbB
(86) International application number: PCT/CN2021/134146
(87) International publication number: WO 2022/111708

(57) **Abstract**

The present invention relates to a deuterated 3-oxo-2,3-dihydropyridazine-4-carboxamide inhibitor, a preparation method therefor, and an application thereof. Specifically, the compound of the present invention has the structure shown in formula (I); also disclosed in the present invention are a preparation method for said compound and the use thereof as an AhR inhibitor; the compound of the present invention has a good selective inhibitory effect on AhR, and has better pharmacodynamics, pharmacokinetic properties, and lower toxic and side effects.

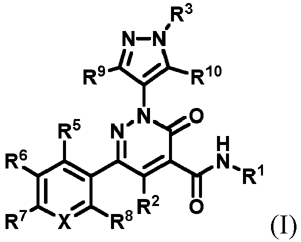

## Description

### TECHNICAL FIELD

The present invention belongs to the field of drugs, and particularly relates to a deuterated 2-arylheterocycle-3-oxo-2,3-dihydropyridazine-4-carboxamide inhibitor, a preparation method therefor and use thereof.

### BACKGROUND

The aryl hydrocarbon receptor (AhR) is a ligand-activated transcription factor involved in the regulation of various cellular processes, including cell proliferation, metabolism, immune regulation, etc. AhR can affect cell signal transduction by interacting with various regulatory and signaling proteins, including PAS heterodimer partner ARNT (aryl hydrocarbon receptor nuclear translocator), chaperone and immune-like proteins (e.g., HSP90), AIP (aryl hydrocarbon receptor-interacting protein), p23, CK2 (casein kinase-2), PKC (protein kinase-C), etc. In addition, AhR also interacts with hormone receptors, hypoxia, NF-KappaB, Rb protein-mediated signaling pathways, MAPK signaling pathways, EGFR signaling pathways, etc. It was discovered in a study that AhR is highly expressed in various tumors, such as lung cancer, colorectal cancer, head and neck squamous cell carcinomas, etc., and can play a key role in the regulation of immunosuppression in tumor microenvironment. A preclinical study showed that mice that are continuously activated spontaneously develop tumors.

AhR is expressed in many cells of the immune system, including dendritic cells (DCs), macrophages, T cells, NK cells, etc. AhR plays an important role in immune regulation: (1) for example, the endogenous AhR ligand may promote the development of Treg cells in TME; (2) AhR promotes the differentiation of Th17 cells through multiple mechanisms; furthermore, AhR regulates the expression of Th17 by binding to the DRE sites on the Th17 promoter; AhR can also cooperate with Stat3 to induce the expression of the Ikaros family member Aiolos (IKZF3), reduce the expression of IL-2 and promote the generation of Th17 cells; (3) the interaction between AhR and c-Maf is critical for the development of mouse and human Tr1 regulatory cells; (4) AhR regulates B-cell differentiation and the like by inhibiting the transcription of B-cell early genes EBF1 and PAX5. Cells in the immune system are constantly exposed to endogenous and exogenous AhR ligands, which can interfere with physiological functions, alter immune homeostasis, and develop into inflammatory diseases, autoimmune diseases, and cancers. The inhibition of AhR can make immunotherapy more effective by reducing immunosuppression.

Exogenous ligands, such as PAH (polycyclic aromatic hydrocarbons), dioxins (e.g., TCDD) and polychlorinated biphenyls, etc., are the main culprits of most toxic reactions. After binding to these environmental toxins, AhR induces metabolic mechanisms, such as cytochrome p450 enzymes, etc. (CYP1A1, CYP1A2, CYP1B1, etc.), to eliminate the environmental toxins. A study showed that exogenous ligands, such as TCDD, etc., that activate AhR have been proved to play a role in many cellular processes, such as embryogenesis, tumorigenesis, inflammation, etc.

In addition to exogenous ligands, AhR can also bind to metabolites of tryptophan degradation. Tryptophan metabolites, such as kynurenine and kynurenic acid, are endogenous AhR ligands that can activate AhR under physiological conditions. The immunosuppressive properties of kynurenine and tryptophan degradation have been fully proved and are involved in cancer-related immunosuppression. In the tryptophan degradation pathway, indoleamine-2,3-dioxygenases 1 and 2 (IDO1/IDO2) and tryptophan-2,3-dioxygenase 2 (TDO2) are responsible for catalyzing the first step and rate-limiting step of tryptophan metabolism. In animal models, the reduction of anti-tumor immune response and the inhibition of IDO can inhibit tumor formation. TDO2 is also strongly expressed in cancer, leading to the production of immunosuppressive kynurenines. In glioma, kynurenine activates AhR, inhibits the anti-tumor immune response by mediating tryptophan degradation downstream, and directly promotes the survival and viability of tumor cells, thus promoting tumor growth. Therefore, the AhR ligands produced by tumor cells act on the tumor cells and the lymphocytes in autocrine and paracrine manners respectively, thereby promoting the tumor growth.

Since AhR target proteins are pathologically associated with various diseases, there is also a need for novel AhR inhibitors for clinical treatment at present. High-selectivity and high-activity AhR inhibitors can more effectively treat cancers and other diseases associated with abnormal AhR mediation and reduce the potential of off-target effect, so there is an urgent clinical demand on the AhR inhibitors.

### SUMMARY

The present invention aims to provide a class of novel compounds with a selective inhibition effect on AhR and/or better pharmacodynamic properties and use thereof.

In a first aspect of the present invention, there is provided a deuterated 3-oxo-2,3-dihydropyridazine-4-carboxamide compound having a structure of general formula (I), or a stereoisomer, a tautomer, a crystalline form, a pharmaceutically acceptable salt, a hydrate, a solvate, or a prodrug thereof:

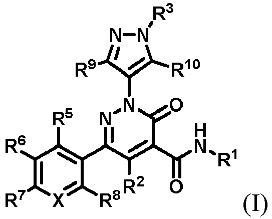

In the formula:
R¹ is selected from the group consisting of the following substituted groups: C₂-C₆ alkyl, C₃-C₁₀ cycloalkyl, and 4- to 10-membered heterocyclyl; wherein the above C₂-C₆ alkyl, C₃-C₁₀ cycloalkyl or 4- to 10-membered heterocyclyl is substituted with at least one hydroxyl group;
R² is selected from the group consisting of hydrogen and deuterium;
R³ is selected from the group consisting of the following substituted or unsubstituted groups: C₁-C₃ alkyl, C₃-C₆ cycloalkyl, and 4- to 6-membered heterocyclyl;
X is selected from the group consisting of N and CR⁴;
R⁴, R⁵, R⁶, R⁸, R⁹, or R¹⁰ is selected from the group consisting of hydrogen and deuterium;
R⁷ is selected from the group consisting of Cl, CF₃, CHF₂, OCF₃, OCHF₂, and N(Me)₂;
wherein the above substitution refers to a substitution by one or more groups selected from the group consisting of: hydrogen, deuterium, C₁-C₁₈ alkyl, deuterated C₁-C₁₈ alkyl, halogenated Ci-Cis alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, nitro, hydroxy, cyano, ester, amino, amido, sulfonamido, and ureido;
the limiting condition is that at least one of R¹, R², R³, R⁴, R⁵, R⁶, R⁷, R⁸, R⁹, and R¹⁰ is deuterium or substituted with a deuterium atom.

In another preferred embodiment, the compound has a structure of general formula (II): 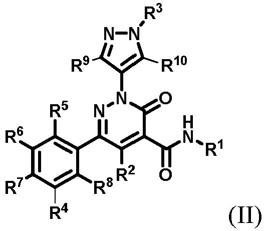 R¹, R², R³, R⁴, R⁵, R⁶, R⁷, R⁸, R⁹, and R¹⁰ are as defined above.

In another preferred embodiment, R¹ is substituted or unsubstituted C₃-C₁₀ cycloalkyl or substituted or unsubstituted saturated 4- to 10-membered heterocyclyl.

In another preferred embodiment, the compound has a structure of general formula (III):

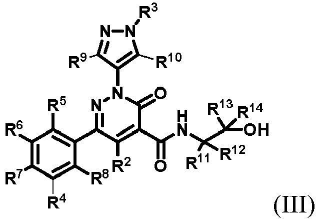

In the formula, R¹¹, R¹², R¹³, and R¹⁴ are each independently selected from the group consisting of the following substituted or unsubstituted groups: H, deuterium, cyano, C₁-C₆ alkyl, C₃-C₆ cycloalkyl, and 4- to 6-membered heterocyclyl; and R², R³, R⁴, R⁵, R⁶, R⁷, R⁸, R⁹, and R¹⁰ are as defined above.

In another preferred embodiment, R¹¹ or R¹² is selected from the group consisting of: D, CN, CH₃, CD₃, CF₃, isopropyl, C₃-C₆ cycloalkyl, and 4- to 6-membered heterocyclyl.

In another preferred embodiment, the carbon atom connected with R¹¹ or R¹² is a chiral carbon atom, the configuration of which is R-type or S-type.

In another preferred embodiment, the compound has a structure of formula (IV):

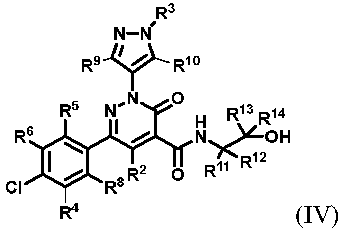

In the formula, R¹¹, R¹², R¹³, and R¹⁴ are each independently selected from the group consisting of the following substituted or unsubstituted groups: H, deuterium, cyano, C₁-C₆ alkyl, C₃-C₆ cycloalkyl, and 4- to 6-membered heterocyclyl; and R², R³, R⁴, R⁵, R⁶, R⁸, R⁹, and R¹⁰ are as defined above.

In another preferred embodiment, R³ is selected from the group consisting of: CH₃ and CD₃.

In another preferred embodiment, R¹³ and R¹⁴ are each independently deuterium.

In another preferred embodiment, R² is D.

In another preferred embodiment, R³ is CD₃.

In another preferred embodiment, R⁹ and R¹⁰ are each independently deuterium.

In another preferred embodiment, R⁴, R⁵, R⁶ and R⁸are each independently deuterium.

In another preferred embodiment, R⁴ and R⁶ are each independently deuterium.

In another preferred embodiment, R⁵ and R⁸ are each independently deuterium.

In another preferred embodiment, the compound is selected from the group consisting of:

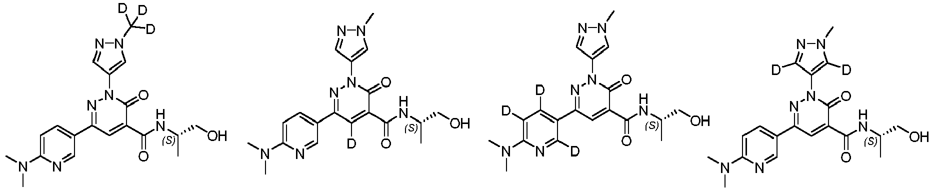

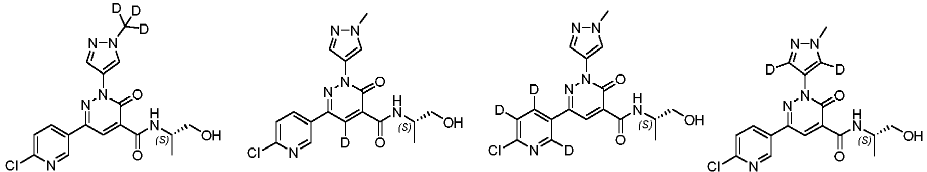

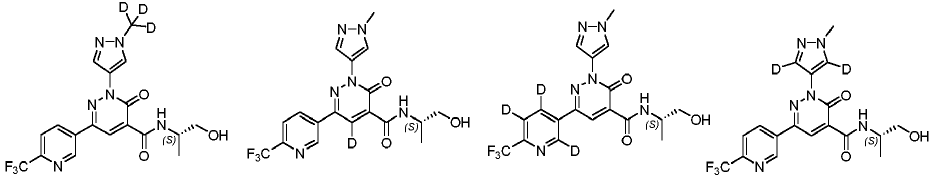

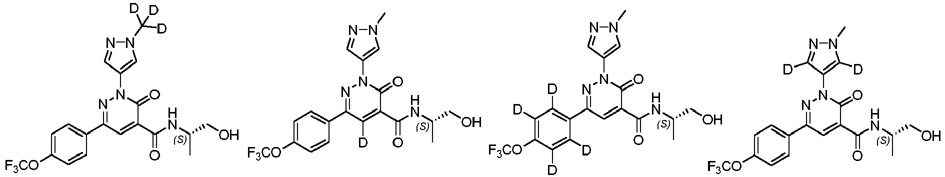

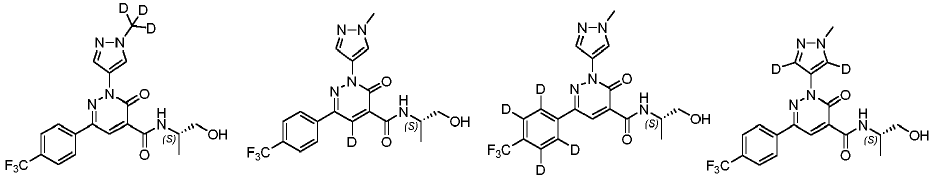

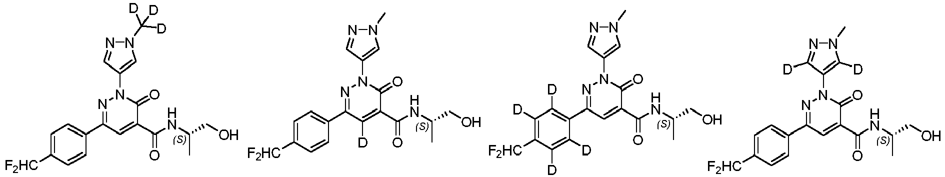

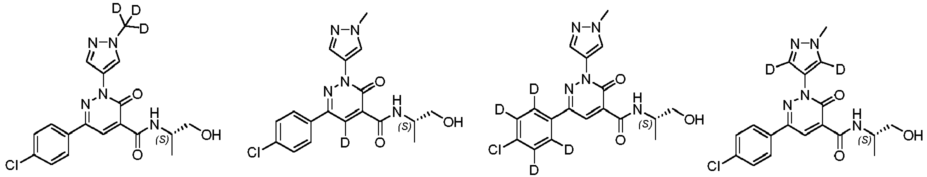

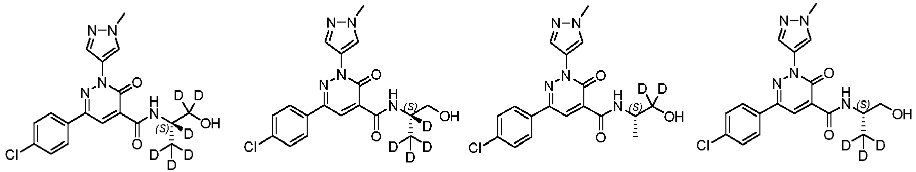

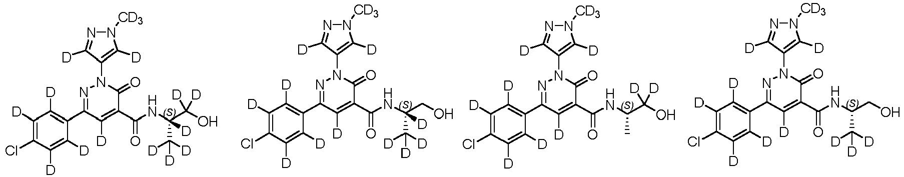

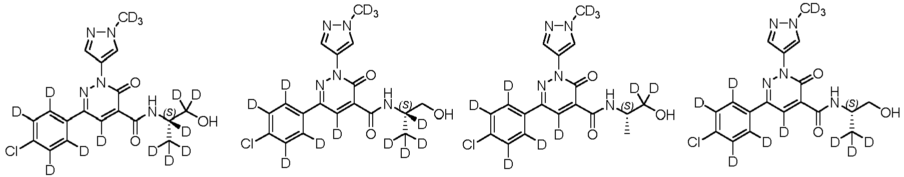

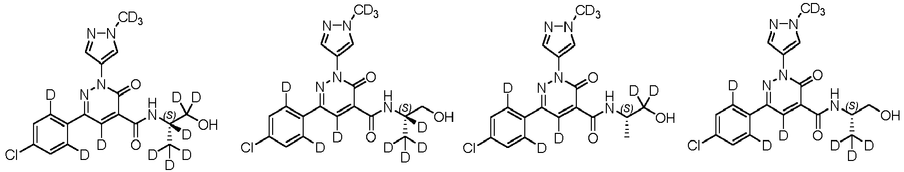

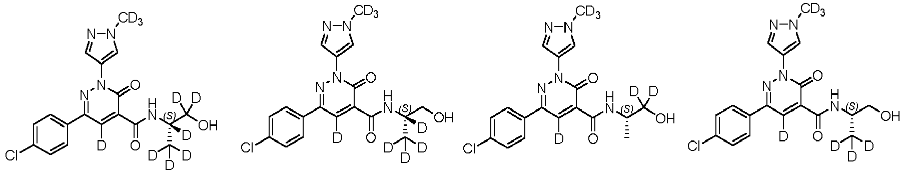

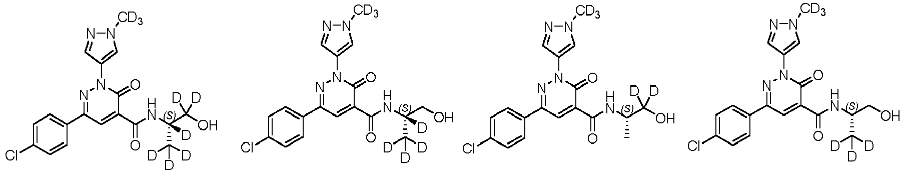

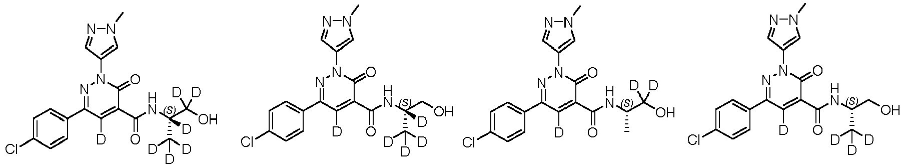

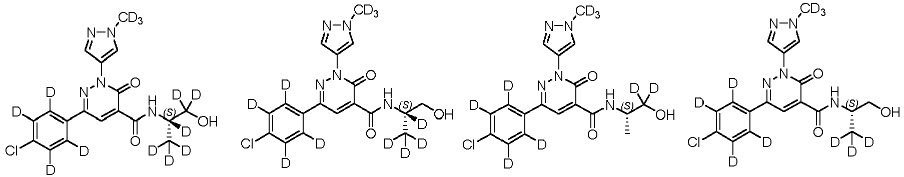

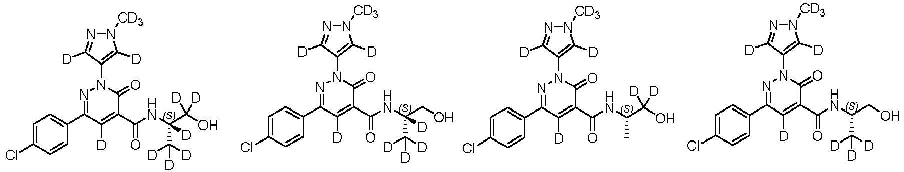

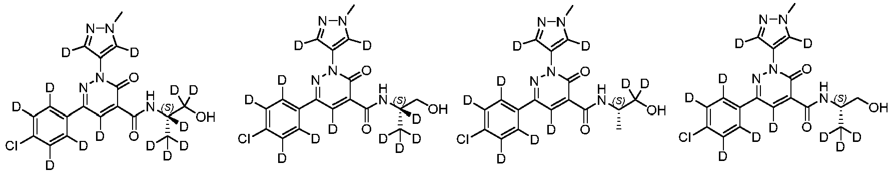

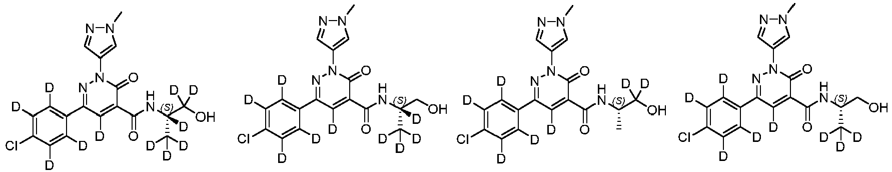

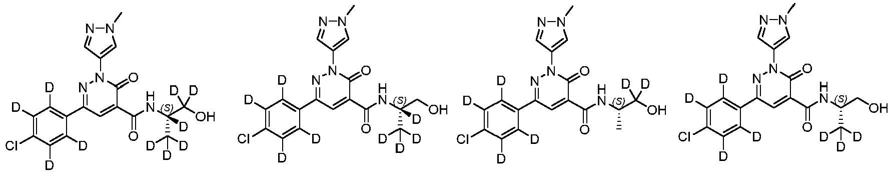

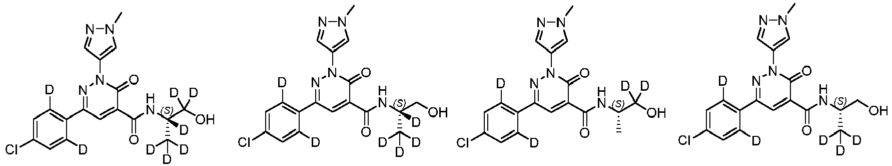

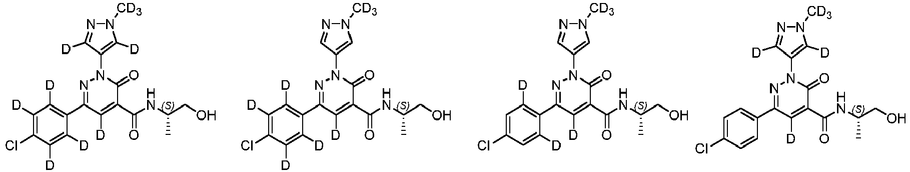

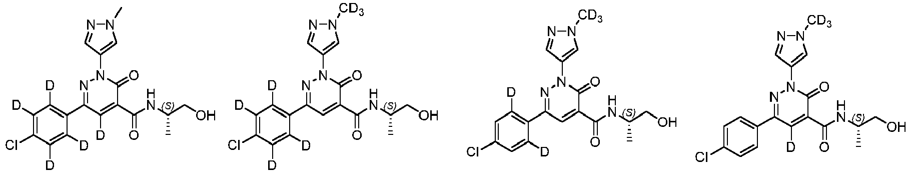

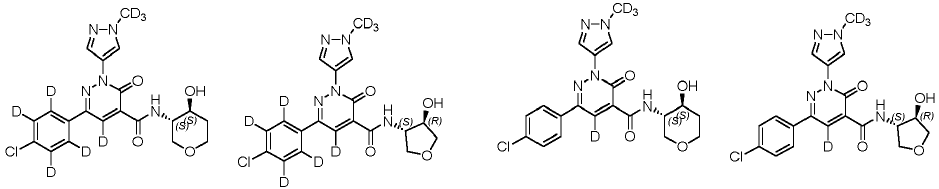

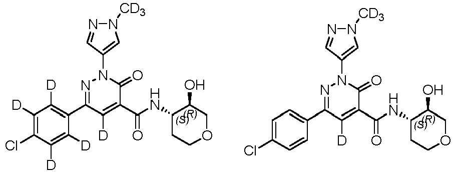

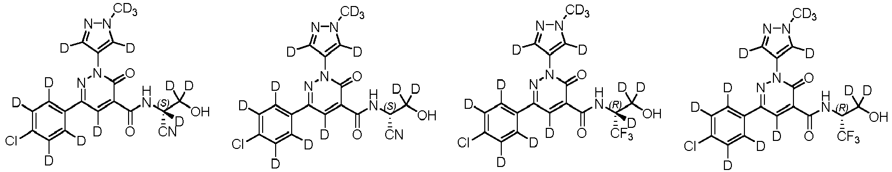

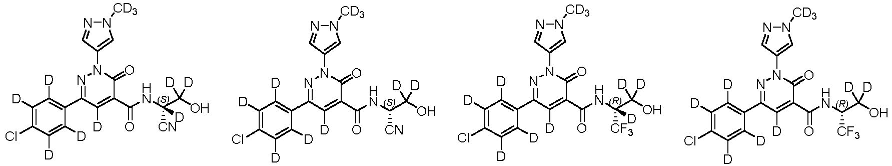

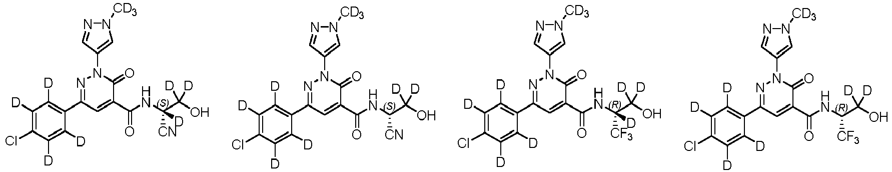

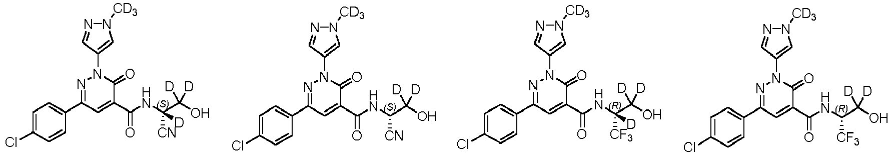

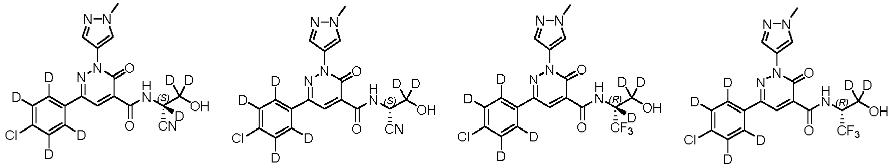

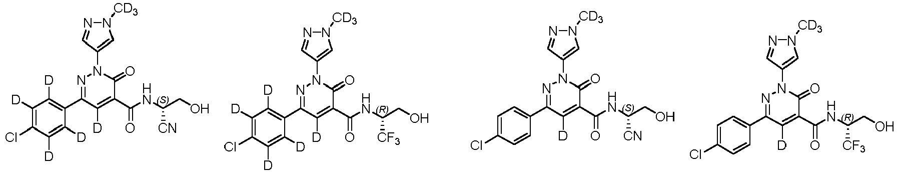

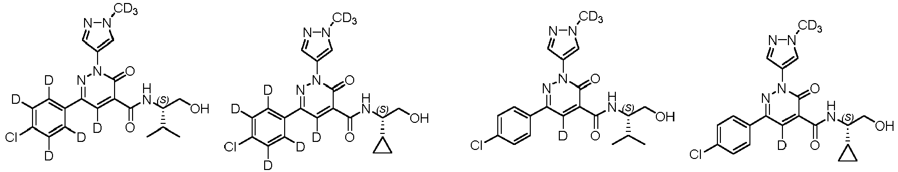

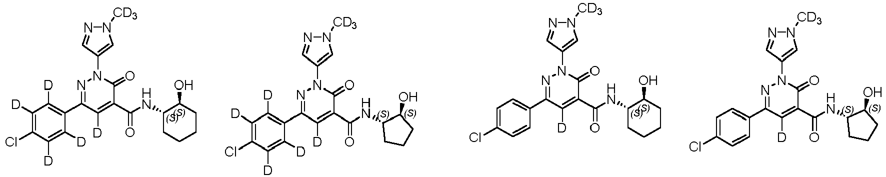

In a second aspect of the present invention, there is provided a method for preparing a deuterated 3-oxo-2,3-dihydropyridazine-4-carboxamide compound having a structure of general formula (I), or a stereoisomer, a tautomer, a crystalline form, a pharmaceutically acceptable salt, a hydrate, a solvate, or a prodrug thereof. The method comprises the steps of: 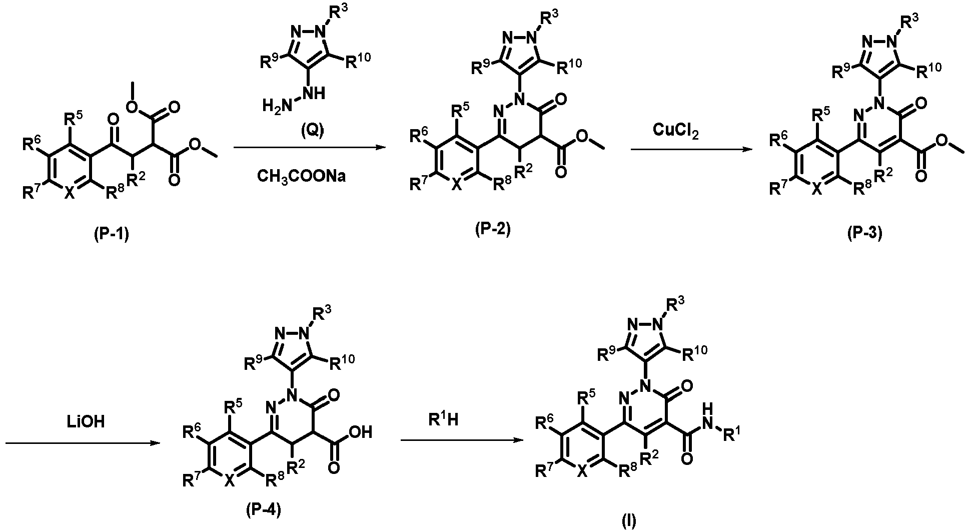
(i) a compound of formula (P-1) reacting with a compound of formula (Q) in the presence of a first base (e.g., sodium acetate) to give a compound of formula (P-2);
(ii) the compound of formula (P-2) carrying out dehydrogenation reaction in the presence of a copper salt (e.g., CuCl₂) to give a compound of formula (P-3);
(iii) hydrolyzing the compound of formula (P-3) in the presence of a second base (e.g., LiOH) to give a compound of formula (P-4);
(iv) the compound of formula (P-4) reacting with an amine (R¹H) to give the compound of formula (I);
in the formula,
R¹, R², R³, R⁵, R⁶, R⁷, R⁸, R⁹, R¹⁰, and X are as defined above.

In a third aspect of the present invention, there is provided a pharmaceutical composition, comprising: i) one or more of the compound having the structure of general formula (I), or the stereoisomer, the tautomer, the crystalline form, the pharmaceutically acceptable salt, the hydrate, the solvate, or the prodrug thereof according to the first aspect of the present invention; and ii) a pharmaceutically acceptable carrier.

In a fourth aspect of the present invention, there is provided use of the deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to the first aspect of the present invention or the pharmaceutical composition according to the third aspect of the present invention in the preparation of a pharmaceutical composition for preventing and/or treating an AhR-mediated disease.

In another preferred embodiment, the disease associated with abnormal AhR mediation is a tumor or a disorder.

In another preferred embodiment, the disease is selected from the group consisting of: lung cancer, breast cancer, prostate cancer, esophageal cancer, colorectal cancer, bone cancer, kidney cancer, stomach cancer, liver cancer, melanoma, lymphoma, leukemia, brain tumor, myeloma, soft tissue sarcoma, pancreatic cancer, and skin cancer.

In another aspect of the present invention, there is provided a method for inhibiting AhR, comprising the step of: administering an effective amount of the compound of general formula (I), or the stereoisomer, the tautomer, the crystalline form, the pharmaceutically acceptable salt, the hydrate, the solvate, or the prodrug thereof according to the first aspect of the present invention or administering the pharmaceutical composition according to the third aspect of the present invention to a patient in need.

It should be understood that within the scope of the present invention, the above various technical features of the present invention and the technical features described in detail below (as in the examples) can be combined with each other to constitute a new or preferred technical solution. Due to limited space, such solutions are not described herein.

### BRIEF DESCRIPTION OF THE DRAWINGS

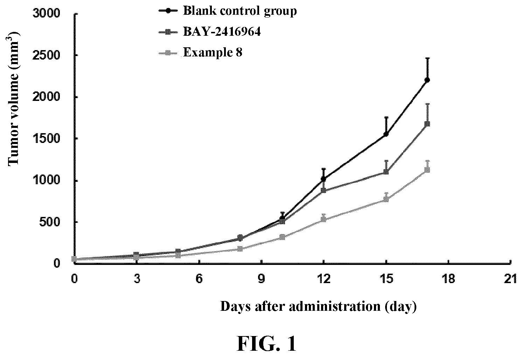

FIG. 1 is a graph of tumor growth in a pharmacodynamic test of anti-tumor activity.

### DETAILED DESCRIPTION

Through a long-term and in-depth study, the inventor unexpectedly discovered a class of novel compounds with a selective inhibition effect on AhR and/or better pharmacodynamic properties. On this basis, the present invention is achieved.

### Terms

In the present invention, unless otherwise specified, the terms used have the general meanings known to those skilled in the art.

The term "alkyl" refers to a linear or branched or cyclic alkyl group containing 1 to 20 carbon atoms (e.g., 1 to 18 carbon atoms, particularly 1 to 18 carbon atoms. Typical "alkyl" groups include methyl, ethyl, propyl, isopropyl, *n*-butyl, *t*-butyl, isobutyl, 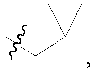 pentyl, isopentyl, heptyl, 4,4-dimethylpentyl, octyl, 2,2,4-trimethylpentyl, nonyl, decyl, undecyl, dodecyl, etc.

The term "C₁-C₁₈ alkyl" refers to a linear or branched or cyclic alkyl group containing 1 to 18 carbon atoms, such as methyl, ethyl, propyl, isopropyl 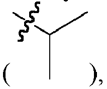 *n*-butyl, *t*-butyl, isobutyl (e.g., 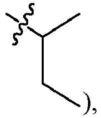 *n*-pentyl, isopentyl, *n*-hexyl, isohexyl, *n*-heptyl, and isoheptyl. "Substituted alkyl" means that one or more positions in an alkyl group are replaced particularly by 1 to 4 substituents, and the substitution may occur at any position. Typical substituents include, but are not limited to, one or more of the following groups: e.g., hydrogen, deuterium, halogen (e.g., monohalo-substituent or polyhalo-substituent, the latter being, for example, trifluoromethyl or alkyl containing Cl₃), cyano, nitro, oxygen (e.g =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Ra, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, and NR_{b}P(=O)₂Rₑ, wherein Rₐ may independently represent hydrogen, deuterium, alkyl, cycloalkyl, alkenyl, cycloalkenyl, alkynyl, heterocyclic ring, or aromatic ring, and R_{b}, R_{c} and R_{d} may independently represent hydrogen, deuterium, alkyl, cycloalkyl, heterocyclic ring, or aromatic ring, or R_{b} and R_{c} can form a heterocyclic ring along with the N atom; and Rₑ may independently represent hydrogen, alkyl, cycloalkyl, alkenyl, cycloalkenyl, alkynyl, heterocyclic ring, or aromatic ring. The above typical substituents, such as alkyl, cycloalkyl, alkenyl, cycloalkenyl, alkynyl, heterocyclic ring, or aromatic ring, may be optional for substitution.

The term "cycloalkyl" refers to a fully saturated cyclic hydrocarbon group including 1 to 4 rings each containing 3 to 8 carbon atoms. "Substituted cycloalkyl" means that one or more positions in a cycloalkyl group are replaced particularly by 1 to 4 substituents, and the substitution may occur at any position. Typical substituents include, but are not limited to, one or more of the following groups: e.g., hydrogen, deuterium, halogen (e.g., monohalo-substituent or polyhalo-substituent, the latter being, for example, trifluoromethyl or alkyl containing Cl₃), cyano, nitro, oxygen (e.g =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, and NR_{b}P(=O)₂Rₑ, wherein Rₐ may independently represent hydrogen, deuterium, alkyl, cycloalkyl, alkenyl, cycloalkenyl, alkynyl, heterocyclic ring, or aromatic ring, and R_{b}, R_{c} and Rₐ may independently represent hydrogen, deuterium, alkyl, cycloalkyl, heterocyclic ring, or aromatic ring, or R_{b} and R_{c} can form a heterocyclic ring along with the N atom; and Rₑ may independently represent hydrogen, deuterium, alkyl, cycloalkyl, alkenyl, cycloalkenyl, alkynyl, heterocyclic ring, or aromatic ring. The above typical substituents may be optional for substitution. Typical substituents also include spiro-, bridged- or fused-ring substituents, particularly spiro-cycloalkyl, spiro-cycloalkenyl, spiro-heterocyclic ring (excluding heteroaromatic ring), bridged-cycloalkyl, bridged-cycloalkenyl, bridged-heterocyclic ring (excluding heteroaromatic ring), fused-cycloalkyl, fused-cycloalkenyl, fused-heterocyclyl, or fused aromatic ring group, and the above cycloalkyl, cycloalkenyl, heterocyclyl and heterocycloaryl groups may be optional for substitution. Any two or more atoms on the rings may be further attached to other cycloalkyl, heterocyclyl, aryl and heteroaryl groups.

The term "heterocyclyl" refers to a fully saturated or partially unsaturated cyclic group (including but not limited to, for example, a 3- to 7-membered monocyclic, 6- to 11-membered bicyclic or 8- to 16-membered tricyclic ring system), with at least one heteroatom being present in the ring having at least one carbon atom. Each heterocyclic ring containing a heteroatom may have 1, 2, 3 or 4 heteroatoms selected from the group consisting of nitrogen atoms, oxygen atoms, and sulfur atoms, among which the nitrogen atoms or the sulfur atoms may be oxidized and the nitrogen atoms may also be quaternized. The heterocyclyl group may be attached to any heteroatom or residue of a carbon atom of the ring or ring system molecule. Typical monocyclic heterocycles include, but are not limited to, azetidinyl, pyrrolidinyl, oxetanyl, pyrazolinyl, imidazolinyl, imidazolidinyl, oxazolidinyl, isoxazolidinyl, thiazolidinyl, isothiazolidinyl, tetrahydrofuryl, piperidinyl, piperazinyl, 2-oxopiperazinyl, 2-oxopiperidinyl, 2-oxopyrrolidinyl, hexahydroazepinyl, 4-piperidinonyl, tetrahydropyranyl, morphinyl, thiomorpholinyl, thiomorpholinosulfoxyl, thiomorpholinosulfuryl, 1,3-dioxanyl, tetrahydro-1,1-dioxythiophene, etc. Polycyclic heterocyclyl groups include spiro-, fused- and bridged-heterocyclyl groups; the spiro-, fused- and bridged-heterocyclyl groups involved are optionally attached to other groups by single bonds or further attached to other cycloalkyl, heterocyclyl, aryl and heteroaryl groups by any two or more atoms on the rings; the heterocyclyl group may be substituted or unsubstituted, and when it is substituted, the substituent is preferably one or more groups independently selected from the group consisting of alkyl, deuterated alkyl, haloalkyl, alkoxy, haloalkoxy, alkenyl, alkynyl, alkylthio, alkylamino, halogen, amino, nitro, hydroxyl, sulfhydryl, cyano, cycloalkyl, heterocyclyl, aryl, heteroaryl, cycloalkylthio, oxo, carboxyl, and carboxylate group, wherein any two or more atoms on the rings may be further attached to other cycloalkyl, heterocyclyl, aryl and heteroaryl groups.

The term "aryl" refers to an aromatic cyclic hydrocarbon group having 1 to 5 rings, particularly monocyclic and bicyclic groups, such as phenyl, biphenyl, or naphthyl. If two or more aromatic rings (bicyclic rings, etc.) are contained, the aromatic rings of the aryl group may be attached by single bonds (e.g., biphenyl) or fused (e.g., naphthalene, anthracene, etc.). "Substituted aryl" means that one or more positions in an aryl group are replaced particularly by 1 to 3 substituents, and the substitution may occur at any position. Typical substituents include, but are not limited to, one or more of the following groups: e.g., hydrogen, deuterium, halogen (e.g., monohalo-substituent or polyhalo-substituent, the latter being, for example, trifluoromethyl or alkyl containing Cl₃), cyano, nitro, oxygen (e.g =O), trifluoromethyl, trifluoromethoxy, cycloalkyl, alkenyl, cycloalkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Ra, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, and NR_{b}P(=O)₂Rₑ, wherein Rₐ may independently represent hydrogen, deuterium, alkyl, cycloalkyl, alkenyl, cycloalkenyl, alkynyl, heterocyclic ring, or aromatic ring, and R_{b}, R_{c} and R_{d} may independently represent hydrogen, deuterium, alkyl, cycloalkyl, heterocyclic ring, or aromatic ring, or R_{b} and R_{c} can form a heterocyclic ring along with the N atom; and Rₑ may independently represent hydrogen, deuterium, alkyl, cycloalkyl, alkenyl, cycloalkenyl, alkynyl, heterocyclic ring, or aromatic ring. The above typical substituents may be optional for substitution. Typical substituents also include fused-ring substituents, particularly fused-cycloalkyl, fused-cycloalkenyl, fused-heterocyclyl, or fused aromatic ring group, and the above cycloalkyl, cycloalkenyl, heterocyclyl and heterocycloaryl groups may be optional for substitution.

The term "heteroaryl" refers to a heteroaromatic system containing 1 to 4 heteroatoms and 5 to 14 ring atoms, wherein the heteroatoms are selected from the group consisting of oxygen, nitrogen, and sulfur. The heteroaryl group is preferably a 5- to 10-membered ring, more preferably a 5- or 6-membered ring, e.g., pyrrolyl, pyrazolyl, imidazolyl, oxazolyl, isoxazolyl, thiazolyl, thiadiazolyl, isothiazolyl, furyl, pyridyl, pyrazinyl, pyrimidinyl, pyridazinyl, triazinyl, triazolyl, tetrazolyl, etc. "Heteroaryl" may be substituted or unsubstituted, and when it is substituted, the substituent is preferably one or more groups independently selected from the group consisting of alkyl, deuterated alkyl, haloalkyl, alkoxy, haloalkoxy, alkenyl, alkynyl, alkylthio, alkylamino, halogen, amino, nitro, hydroxyl, sulfhydryl, cyano, cycloalkyl, heterocyclyl, aryl, heteroaryl, cycloalkylthio, oxo, carboxyl, and carboxylate group.

The term "halogen" or "halo-" refers to chlorine, bromine, fluorine, and iodine.

The term "halo" refers to a substitution by halogen.

The term "deuterated" refers to a substitution by deuterium.

The term "hydroxyl" refers to a group with a structure OH.

The term "nitro" refers to a group with a structure NO₂.

The term "cyano" refers to a group with a structure CN.

The term "ester" refers to a group with a structure -COOR, wherein R represents hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring.

The term "amino" refers to a group with a structure -NRR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R and R' may be the same or different in a dialkylamine moiety.

The term "amido" refers to a group with a structure -CONRR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R and R' may be the same or different in a dialkylamine moiety.

The term "sulfonamido" refers to a group with a structure -SO₂NRR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R and R' may be the same or different in a dialkylamine moiety.

The term "ureido" refers to a group with a structure -NRCONR'R", wherein R, R' and R" may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R, R' and R" may be the same or different in a dialkylamine moiety.

The term "alkylaminoalkyl" refers to a group with a structure -RNHR', wherein R and R' may independently represent hydrogen, alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R and R' may be the same or different.

The term "dialkylaminoalkyl" refers to a group with a structure -RNHR'R", wherein R, R' and R" may independently represent alkyl or substituted alkyl, cycloalkyl or substituted cycloalkyl, cycloalkenyl or substituted cycloalkenyl, aryl or substituted aryl, or a heterocyclic ring or a substituted heterocyclic ring, as defined above. R, R' and R" may be the same or different in a dialkylamine moiety.

The term "heterocyclylalkyl" refers to a group with a structure -RR', wherein R may independently represent alkyl or substituted alkyl, cycloalkyl or substituted cycloalkyl, cycloalkenyl or substituted cycloalkenyl, or aryl or substituted aryl, and R' represents a heterocyclic ring or a substituted heterocyclic ring.

In the present invention, the term "substitute" means that one or more hydrogen atoms on a specific group are substituted with specific substituents. The specific substituents are those described correspondingly in the preceding text or those presented in each example. Unless otherwise specified, a substituted group may have a substituent selected from a specific group at any substitutable positions of the group, and the substituent may be the same or different at each position. It should be understood by those skilled in the art that the combinations of substituents contemplated by the present invention are those stable or chemically available. The substituents are for example (but not limited to): halogen, hydroxy, cyano, carboxyl (-COOH), C₁-C₆ alkyl, C₂-C₆ alkenyl, C₂-C₆ alkynyl, C₃-C₈ cycloalkyl, 3- to 12-membered heterocyclyl, aryl, heteroaryl, C₁-C₈ formyl, C₂-C₁₀ acyl, C₂-C₁₀ ester, amino, C₁-C₆ alkoxy, C₁-C₁₀ sulfonyl, C₁-C₆ ureido, etc.

Unless otherwise specified, it is assumed that any heteroatom that is not in a sufficient valence state has sufficient hydrogen atoms to supplement its valence state.

When the substituents are non-terminal substituents, they are subunits of the corresponding groups, e.g., alkyl corresponding to alkylene, cycloalkyl corresponding to cycloalkylene, heterocyclyl corresponding to heterocyclylene, and alkoxy corresponding to alkyleneoxy.

### Active Ingredient

The present invention provides a deuterated 3-oxo-2,3-dihydropyridazine-4-carboxamide compound having a structure of general formula (I), or a stereoisomer, a tautomer, a crystalline form, a pharmaceutically acceptable salt, a hydrate, a solvate, or a prodrug thereof:

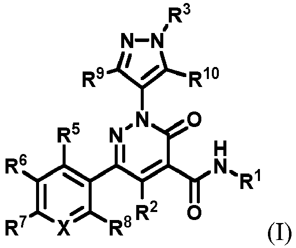

In the formula:
R¹ is selected from the group consisting of the following substituted groups: C₂-C₆ alkyl, C₃-C₁₀ cycloalkyl, and 4- to 10-membered heterocyclyl; wherein the above C₂-C₆ alkyl, C₃-C₁₀ cycloalkyl or 4- to 10-membered heterocyclyl is substituted with at least one hydroxyl group;
R² is selected from the group consisting of hydrogen and deuterium;
R³ is selected from the group consisting of the following substituted or unsubstituted groups: C₁-C₃ alkyl, C₃-C₆ cycloalkyl, and 4- to 6-membered heterocyclyl;
X is selected from the group consisting of N and CR⁴;
R⁴, R⁵, R⁶, R⁸, R⁹, or R¹⁰ is selected from the group consisting of hydrogen and deuterium;
R⁷ is selected from the group consisting of Cl, CF₃, CHF₂, OCF₃, OCHF₂, and N(Me)₂;
wherein the above substitution refers to a substitution by one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, nitro, hydroxy, cyano, ester, amino, amido, sulfonamido, and ureido;
the limiting condition is that at least one of R¹, R², R³, R⁴, R⁵, R⁶, R⁷, R⁸, R⁹, and R¹⁰ is deuterium or substituted with a deuterium atom.

The salts which may be formed by the compound in the present invention are also within the scope of the present invention. Unless otherwise specified, the compound of the present invention should be understood to include salts thereof. The term "salt" used herein refers to an acidic or basic salt formed from an inorganic or organic acid and a base. In addition, when the compound of the present invention contains a basic moiety, it includes, but is not limited to, pyridine or imidazole; when the compound of the present invention contains an acidic moiety, it includes, but is not limited to, carboxylic acid; and zwitter-ions ("inner salts") that may be formed are included within the scope of the term "salt". Pharmaceutically acceptable (i.e., non-toxic, physiologically acceptable) salts are preferred, although other salts are useful in, for example, isolation or purification step of the preparation process. The compound of the present invention may form salts. For example, the compound (I) reacts with an amount (e.g., an equivalent amount) of acid or base, and a salt is isolated by salting out from a medium or by lyophilization in aqueous solution.

The compound of the present invention may contain basic moieties, including but not limited to amine or pyridine or imidazole rings, which may form salts along with organic or inorganic acids. Typical salts which may be formed by acids include acetate (e.g., formed with acetic acid or trihaloacetic acid, such as trifluoroacetic acid), adipate, alginate, ascorbate, aspartate, benzoate, benzenesulfonate, bisulfate, borate, butyrate, citrate, camphorate, camphorsulfonate, cyclopentanepropionate, diglycolate, laurylsulfate, ethanesulfonate, fumarate, glucoheptonate, glycerophosphate, hemisulfate, heptanoate, caproate, hydrochloride, hydrobromide, hydriodate, hydroxyethanesulfonate (e.g., 2-hydroxyethanesulfonate), lactate, maleate, methanesulfonate, naphthalenesulfonate (e.g., 2-naphthalenesulfonate), nicotinate, nitrate, oxalate, pectate, persulfate, phenpropionate (e.g., 3-phenpropionate), phosphate, picrate, pivalate, propionate, salicylate, succinate, sulfate (e.g., formed with sulfuric acid), sulfonate, tartrate, thiocyanate, tosylate (e.g., *p-*toluenesulfonate), dodecanoate, and the like.

Certain compounds of the present invention may contain acidic moieties, including but not limited to carboxylic acids, which may form salts along with various organic or inorganic bases. Typical salts formed by bases include ammonium salts, alkali metal salts, such as sodium salt, lithium salt, and potassium salt, alkaline earth metal salts, such as calcium salt and magnesium salt, salts formed with organic bases (e.g., organic amines), such as benzathine, dicyclohexylamine, hydrabamine (a salt formed with *N, N*-di(dehydroabietyl)ethylenediamine), *N*-methyl-D-glucamine, *N*-methyl-D-glucamide, and *t*-butylamine, and salts formed with amino acids (e.g., arginine, lysine, etc.). The basic nitrogen-containing groups may be combined with halide quaternary ammonium salts, such as small-molecular alkyl halides (e.g., methyl, ethyl, propyl, and butyl chlorides, methyl, ethyl, propyl, and butyl bromides, and methyl, ethyl, propyl, and butyl iodides), dialkyl sulfates (e.g., dimethyl, diethyl, dibutyl, and diamyl sulfates), long-chain halides (e.g., decyl, dodecyl, tetradecyl, and tetradecyl chlorides, decyl, dodecyl, tetradecyl, and tetradecyl bromides, and decyl, dodecyl, tetradecyl, and tetradecyl iodides), aralkyl halides (e.g., benzyl and phenyl bromides), etc.

Prodrugs and solvates of the compounds of the present invention also fall within the scope of the present invention. The term "prodrug" used herein refers to a compound that undergoes chemical transformation through a metabolic or chemical process to give a compound, salt, or solvate of the present invention when used in the treatment of a related disease. The compounds of the present invention include solvates, such as hydrates.

The compounds, salts or solvates of the present invention may exist in tautomeric forms (e.g., amides and imine ethers). All these tautomers are part of the present invention.

Stereoisomers of all the compounds (e.g., those asymmetric carbon atoms which may exist due to various substitutions), including enantiomeric and diastereomeric forms thereof, shall fall within the scope of the present invention. The independent stereoisomers of the compounds of the present invention may not be present in combination with other isomers (e.g., having special activity as a pure or substantially pure optical isomer), or may be present as a mixture, such as a racemate, or as a mixture formed with all or part of the other stereoisomers. The chiral center of the present invention has two configurations, S or R, as defined by the recommendation of the International Union of Pure and Applied Chemistry (IUPAC) in 1974. The racemic forms may be resolved by physical methods, such as fractional crystallization, separation or crystallization of derived diastereoisomers, or chiral column chromatography. Individual optical isomers may be obtained from racemates by suitable methods, including but not limited to conventional methods, such as salt formation with an optically active acid followed by crystallization.

The content, by weight, of the compound of the present invention, which is obtained by preparation, separation and then purification, is equal to or greater than 90%, e.g., is equal to or greater than 95%, or is equal to or greater than 99% ("very pure" compound), as listed in the text description. Herein, such "very pure" compounds of the present invention are also part of the present invention.

All configurational isomers of the compounds of the present invention fall within the scope of the present invention, whether in mixture, pure or very pure form. The definition of the compound of the present invention includes cis (Z) and trans (E) olefin isomers, as well as cis and trans isomers of carbocyclic and heterocyclic rings.

Throughout the specification, groups and substituents can be selected to provide stable moieties and compounds.

Specific functional groups and definitions of chemical terms are described in detail below. For the present invention, the chemical elements are consistent with those defined in Periodic Table of the Elements, CAS version, Handbook of Chemistry and Physics, 75th Ed. The definitions of specific functional groups are also described herein. In addition, the basic principles of organic chemistry, specific functional groups and reactivity are also explained in *"*Organic Chemistry", Thomas Sorrell, University Science Books, Sausalito: 1999, the entire contents of which are incorporated by reference.

Certain compounds of the present invention may exist in the form of specific geometric isomers or stereisomers. The present invention encompasses all the compounds, including cis and trans isomers, R and S enantiomers, diastereomers, (D) isomers, (L) isomers, racemic mixtures, and other mixtures. In addition, the asymmetric carbon atoms may represent substituents, such as alkyl groups. All the isomers and mixtures thereof are encompassed by the present invention.

According to the present invention, the ratio of isomers in a mixture of the isomers may be varied. For example, in a mixture of only two isomers there may be the following combinations: 50:50, 60:40, 70:30, 80:20, 90:10, 95:5, 96:4, 97:3, 98:2, 99:1, or 100:0, ratios of all isomers being within the scope of the present invention. Similar ratios which are easily understood by those of ordinary skill in the art, as well as ratios in mixtures of more complex isomers, are also within the scope of the present invention.

The present invention also includes isotopically labeled compounds, equivalent to the disclosure of the original compounds herein. In fact, however, it usually occurs that one or more atoms are substituted with atoms with different atomic weights or mass numbers. Examples of isotopes that may be listed as the compounds of the present invention include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl. The compounds or the enantiomers, diastereomers, isomers or pharmaceutically acceptable salts or solvates (containing the isotopes of the above compounds or other isotope atoms) thereof in the present invention are all within the scope of the present invention. Certain isotopically labeled compounds of the present invention, e.g., including radioisotopes of ³H and ¹⁴C, are also useful in an tissue distribution study of drugs and substrates. Tritium (i.e., ³H) and carbon-14 (i.e.,¹⁴C) are easy to prepare and detect. They are the first choice among isotopes. In addition, heavier isotope substitutions such as deuterium, (i.e., ²H) have advantages (e.g., increased half-life or reduced dose in vivo) in some therapies due to its good metabolic stability and hence may be preferred in some cases. The isotopically labeled compounds may be prepared by the general method of replacing a readily available isotopically labeled reagent with a non-isotopically labeled reagent according to the solutions disclosed in the examples.

If it is desired to design the synthesis of a specific enantiomer of a compound of the present invention, it may be prepared by asymmetric synthesis or derivatised with a chiral auxiliary, the resulting diastereomeric mixture is separated, and the chiral auxiliary is then removed to give a pure enantiomer. In addition, if the molecule contains a basic functional group (such as amino acid) or an acidic functional group (such as carboxyl), a diastereomer salt can be formed with an appropriate optically active acid or base, and separation is carried out by a conventional means such as fractional crystallization or chromatography to give a pure enantiomer.

As described herein, the compounds of the present invention may be substituted with any number of substituents or functional groups to expand the scope of the present invention. Generally, the term "substitute", whether appearing before or after the term "optionally", means that the hydrogen radical is replaced by a substituent with a specific structure in a general formula containing the substituent in a formulation of the present invention. When a plurality of positions in a specific structure are substituted with a plurality of specific substituents, each position of the substituents may be the same or different. The term "substitute" used herein includes substitution by all allowed organic compounds. In a broad sense, allowed substituents include acyclic, cyclic, branched, unbranched, carbocyclic, heterocyclic, aromatic, and nonaromatic organic compounds. In the present invention, for example, the heteroatom nitrogen may have a hydrogen substituent or any allowed organic compound mentioned above to supplement its valence state. In addition, the present invention is not intended to limit organic compounds allowed for substitution in any way. The present invention believes that the combination of substituents and variable groups is excellent in the treatment of diseases, such as infectious diseases or proliferative diseases, in the form of stable compounds. The term "stable" used herein means that a compound is stable enough to maintain the structural integrity of the compound for a long time when detected long enough, preferably maintaining efficacy long enough, for the above purpose herein.

Metabolites of the compounds and the pharmaceutically acceptable salts thereof involved in the present application, as well as prodrugs that can be transformed into the structures of the compounds and the pharmaceutically acceptable salts thereof involved in the present application in vivo, are also included in the claims of the present application.

### Preparation Method

Methods for preparing the compound having the structure of formula (I) disclosed herein are described in more details below, but these specific methods do not limit the present invention in any way. The compound of the present invention can also be conveniently prepared by optionally combining various synthetic methods described herein or known in the art, and such combinations can be easily implemented by those skilled in the art to which the present invention pertains.

Typically, the process for preparing the compound of the present invention is as follows, in which the materials and reagents used are commercially available unless otherwise specified. 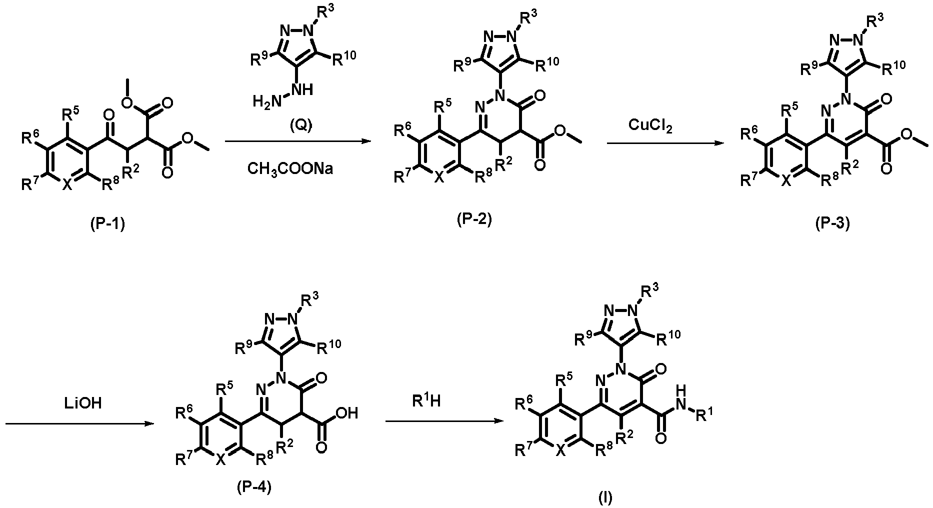
(i) a compound of formula (P-1) reacting with a compound of formula (Q) in the presence of a first base (e.g., sodium acetate) to give a compound of formula (P-2);
(ii) the compound of formula (P-2) carrying out dehydrogenation reaction in the presence of a copper salt (e.g., CuCl₂) to give a compound of formula (P-3);
(iii) hydrolyzing the compound of formula (P-3) in the presence of a second base (e.g., LiOH) to give a compound of formula (P-4);
(iv) the compound of formula (P-4) reacting with an amine (R¹H) to give the compound of formula (I);
in the formula, R¹, R², R³, R⁵, R⁶, R⁷, R⁸, R⁹, R¹⁰, and X are as defined above.

### Pharmaceutical Composition and Administration Route

The pharmaceutical composition disclosed herein is used for preventing and/or treating the following diseases: inflammation, cancer, cardiovascular diseases, infection, immunological diseases, and metabolic diseases.

The compound of general formula (I) may be administered in combination with other drugs known to treat or ameliorate similar conditions. During administration in combination, the administration route and dose of the original drug may be kept unchanged, and the compound of formula I may be administered simultaneously or subsequently. When the compound of formula I is administered simultaneously with one or more other drugs, a pharmaceutical composition containing one or more known drugs and the compound of formula I may be preferably used. The administration of the drugs in combination also includes the administration of the compound of formula I and one or more other known drugs in overlapping time periods. When the compound of formula I is administered in combination with one or more other drugs, the doses of the compound of formula I or the known drugs may be lower than those of them administered alone.

Drugs or active ingredients that may be administered in combination with the compound of general formula (I) include, but are not limited to: PD-1 inhibitors (e.g., nivolumab, pembrolizumab, pidilizumab, cemiplimab, JS-001, SHR-120, BGB-A317, IBI-308, GLS-010, GB-226, STW204, HX008, HLX10, BAT1306, AK105, LZM009, or biologically similar drugs thereof), PD-L1 inhibitors (e.g., durvalumab, atezolizumab, avelumab, CS1001, KN035, HLX20, SHR-1316, BGB-A333, JS003, CS1003, KL-A167, F520, GR1405, MSB2311, or biologically similar drugs thereof), CD20 antibodies (e.g. rituximab, obinutuzumab, ofatumumab, veltuzumab, tositumomab, 131I-tositumomab, ibritumomab, 90Y-ibritumomab, 90In-ibritumomab, ibritumomab tiuxetan, etc.), CD47 antibodies (e.g. Hu5F9-G4, CC-90002, TTI-621, TTI-622, OSE-172, SRF-231, ALX-148, NI-1701, SHR-1603, IBI188, and IMM01), ALK inhibitors (e.g. Ceritinib, Alectinib, Brigatinib, Lorlatinib, and alkotinib), PI3K inhibitors (e.g. Idelalisib, Duvelisib, Dactolisib, Taselisib, Bimiralisib, Omipalisib, Buparlisib, etc.), BTK inhibitors (e.g. Ibrutinib , Tirabrutinib, Acalabrutinib, Zanubrutinib, Vecabrutinib, etc.), EGFR inhibitors (e.g. Afatinib, Gefitinib, Erlotinib , Lapatinib, Dacomitinib, Icotinib, Canertinib, Sapitinib, Naquotinib, Pyrotinib, Rociletinib, Osimertinib, etc.), VEGFR inhibitors (e.g. Sorafenib, Pazopanib, Regorafenib, Sitravatinib, Ningetinib, Cabozantinib, Sunitinib, Donafenib, etc.), HDAC inhibitors (e.g. Givinostat, Tucidinostat, Vorinostat, Fimepinostat, Droxinostat, Entinostat, Dacinostat, Quisinostat, Tacedinaline, etc.), CDK inhibitors (e.g. Palbociclib, Ribociclib, Abemaciclib, Milciclib, Trilaciclib, Lerociclib, etc.), MEK inhibitors (e.g. Selumetinib (AZD6244), Trametinib (GSK1120212), PD0325901, U0126, Pimasertib (AS-703026), PD184352 (CI-1040), etc.), mTOR inhibitors (e.g. Vistusertib, etc.), SHP2 inhibitors (e.g. RMC-4630, JAB-3068, TNO155, etc.), or combinations thereof.

Dosage forms of the pharmaceutical composition of the present invention include (but are not limited to): injections, tablets, capsules, aerosols, suppositories, pellicles, dripping pills, topical liniment, controlled-release or sustained-release or nanometer preparations.

The pharmaceutical composition of the present invention contains the compound of the present invention or a pharmacologically acceptable salt thereof and a pharmacologically acceptable excipient or carrier in a safe and effective amount range, wherein the "safe and effective amount" means that the amount of the compound is sufficient to significantly improve the condition without causing serious side effects. Generally, the pharmaceutical composition contains 1-2000 mg of the compound of the present invention per dose, more preferably 10-1000 mg of the compound of the present invention per dose. Preferably, the "dose" is a capsule or tablet.

"Pharmaceutically acceptable carrier" refers to one or more compatible solid or liquid fillers or gel substances which are suitable for human use and must be of sufficient purity and sufficiently low toxicity. "Compatible" herein means that the components of the composition are capable of intermixing with the compound of the present invention and with each other, without significantly diminishing the pharmaceutical efficacy of the compound. Examples of the pharmaceutically acceptable carriers include cellulose and derivatives thereof (e.g., sodium carboxymethylcellulose, sodium ethylcellulose, cellulose acetate, etc.), gelatin, talc, solid lubricants (e.g., stearic acid and magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifiers (e.g., Tween^{®}), wetting agents (e.g., sodium dodecyl sulfate), colorants, flavoring agents, stabilizers, antioxidants, preservatives, pyrogen-free water, etc.

The administration routes of the compound or pharmaceutical composition of the present invention are not specifically limited, typically including (but not limited to): oral administration, intratumoral administration, rectal administration, parenteral (intravenous, intramuscular or subcutaneous) administration, and topical administration.

Solid dosage forms for oral administration include capsules, tablets, pills, pulvises, and granules. In these solid dosage forms, the active compound is mixed with at least one conventional inert excipient (or carrier), such as sodium citrate or calcium phosphate, or mixed with the following ingredients: (a) fillers or solubilizers, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato starch or tapioca starch, alginic acid, certain complex silicates, and sodium carbonate; (e) slow-dissolving agents, such as paraffin; (f) absorption accelerators, such as a quaternary amine compound; (g) wetting agents, such as cetyl alcohol and glycerol monostearate; (h) adsorbents, such as kaolin; (i) lubricants, such as talc, calcium stearate, magnesium stearate, solid polyethylene glycol, sodium dodecyl sulfate, or mixtures thereof. In capsules, tablets and pills, the dosage forms may also comprise buffers.

Solid dosage forms, such as tablets, dragees, capsules, pills, and granules, can be prepared using coatings and shells, such as enteric coatings and other materials well known in the art. They may comprise opacifying agents, and the active compound or compound in such a composition may be released in a certain part of the digestive tract in a delayed manner. Examples of embedding components which can be used are polymeric substances and wax-based substances. If necessary, the active compound can also be in microcapsule form with one or more of the above-mentioned excipients.

Liquid dosage forms for oral administration include pharmaceutically acceptable emulsions, solutions, suspensions, syrups, and tinctures. Besides the active compound, the liquid dosage forms may comprise inert diluents conventionally used in the art, such as water or other solvents, solubilizers and emulsifiers, e.g., ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil and sesame oil, or mixtures of these substances, etc.

Besides such inert diluents, the composition may also comprise adjuvants, such as wetting agents, emulsifiers, suspending agents, sweeteners, flavoring agents, and perfuming agents.

Besides the active compound, the suspensions may comprise suspending agents, e.g., ethoxylated isostearyl alcohols, polyoxyethylene sorbitol and sorbitan esters, microcrystalline cellulose, aluminum methylate and agar, or mixtures of these substances, etc.

The compositions for parenteral injection may comprise physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents or excipients include water, ethanol, polyols, and suitable mixtures thereof.

Dosage forms of the compound of the present invention for topical administration include ointments, pulvises, patches, sprays, and inhalants. The active ingredient is mixed under sterile conditions with a physiologically acceptable carrier and any preservatives, buffers, or propellants which may be needed if necessary.

The therapeutic method of the present invention may be applied alone or in combination with other therapeutic means or therapeutic drugs.

When the pharmaceutical composition is used, a safe and effective amount of the compound of the present invention is administered to a mammal (e.g., a human) to be treated, wherein the administration dose is a pharmaceutically effective administration dose. For a human weighing 60 kg, the daily administration dose is usually 1-2000 mg, preferably 50-1000 mg. In determining a specific dose, such factors as the route of administration, the health condition of the patient and the like will also be considered, which are well known to skilled physicians.

The present invention also provides a method for preparing the pharmaceutical composition, which comprises the step of: mixing a pharmaceutically acceptable carrier with the compound of general formula (I) of the present invention or the crystalline form, pharmaceutically acceptable salt, hydrate or solvate thereof to form the pharmaceutical composition.

The present invention also provides a treatment method, which comprises the step of: administering to a subject to be treated the compound of general formula (I) of the present invention or the crystalline form, pharmaceutically acceptable salt, hydrate or solvate thereof, or the pharmaceutical composition of the present invention for selectively inhibiting AhR.

**Compared with the prior art, the present invention mainly has the following advantages:**
(1) The compound has a good selective inhibition effect on AhR;
(2) The compound has better in-vivo and in-vitro pharmacodynamic and pharmacokinetic properties, and lower toxic and side effects.

The present invention will be further illustrated with reference to the following specific examples. It should be understood that these examples are merely intended to illustrate the present invention rather than limit the scope of the present invention. In the following examples, study methods without specific conditions were generally in accordance with conventional conditions, such as conditions described in Sambrook, et al., Molecular Cloning: Laboratory Manual (New York: Cold Spring Harbor Laboratory Press, 1989), or conditions recommended by the manufacturer. Unless otherwise specified, percentages and parts are by weight.

Unless otherwise defined, all technical and scientific terms used herein have the same meaning as commonly understood by those skilled in the art. In addition, any methods and materials similar or equivalent to those described herein can be applied in the methods of the present invention. The preferred embodiments and materials described herein are merely intended for demonstration.

The compound structure of the present invention is determined by nuclear magnetic resonance (NMR) and liquid chromatography-mass spectrometry (LC-MS).

NMR is determined using a Bruker AVANCE-400 nuclear magnetic resonance instrument, with deuterated dimethylsulfoxide (DMSO-d₆), deuterated acetone (CD₃COCD₃), deuterated chloroform (CDCl₃), deuterated methanol (CD₃OD) and the like as determination solvents and tetramethylsilane (TMS) as an internal standard, and chemical shifts are measured in unit of 10⁻⁶ (ppm).

Liquid chromatography-mass spectrometry (LC-MS) is determined using a Waters SQD2 mass spectrometer. HPLC determination is performed using an Agilent 1100 high-pressure chromatograph (Microsorb 5 micron C18 100 × 3.0 mm column).

Qingdao GF254 silica gel plate is adopted as a thin-layer chromatography (TLC) silica gel plate, 0.15-0.20 mm adopted for TLC and 0.4-0.5 mm adopted for the preparation of thin-layer chromatography. Qingdao silica gel with 200-300 meshes is generally adopted as a carrier in column chromatography.

Starting materials in the examples of the present invention are known and commercially available, or may be synthesized by adopting or according to literatures reported in the art.

Unless otherwise specified, all the reactions in the present invention are carried out by continuous magnetic stirring under the protection of dry inert gas (such as nitrogen or argon), and all the reaction temperature are in Celsius.

### Examples

### Example 1: Preparation of Intermediates

### Example 1-1: Synthesis of 4-hydrazino-1-(methyl-d3)-1H-pyrazole

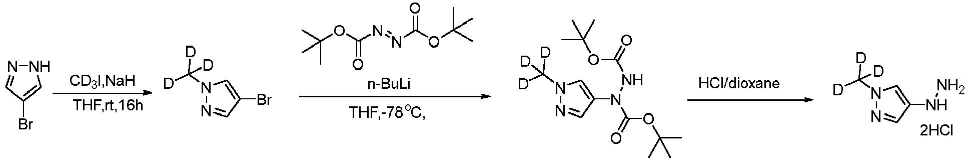

### Step 1: Preparation of 4-bromo-1-(methyl-d3)-1H-pyrazole

4-bromopyrazole (3.90 g, 26.50 mmol) was dissolved in anhydrous tetrahydrofuran (39 mL), and the solution was cooled to 5 °C in an ice-water bath and then added with sodium hydrogen (wt%: 60%, 1.22 g, 30.50 mmol) in batches. After the addition, the reaction solution was warmed to room temperature, stirred for 30 min, then cooled to 5 °C and added with deuterated iodomethane (5.0 g, 34.49 mmol). The resulting reaction solution was stirred at room temperature for 16 h and then concentrated under vacuum to become dry. The residue was slurried with methyl t-butyl ether (30 mL) and then filtered, the filter cake was rinsed with methyl *t*-butyl ether, and the filtrate was concentrated to give the target product (3.5 g). The product was directly used in the next reaction without purification.

### Step 2: Preparation of Di-t-butyl 1-(1-(methyl-d3)-1H-pyrazole-4-yl)hydrazino-1,2-dicarboxylate

4-bromo-1-(methyl-*d*3)-1*H*-pyrazole (3.5 g, 21.3 mmol) was added into THF (50 mL), the solution was cooled to -65 °C, and an *n*-butyllithium solution (2.5 M, 10 mL, 25.56 mmol) was then added dropwise. After the addition, the reaction solution was stirred for 0.5 h while the temperature was kept, the solution (50 mL) of di-*t*-butyl (E)-diazene-1,2-dicarboxylate (5.2 g, 22.4 mmol) in THF was then added dropwise into a reaction flask, and the temperature was controlled to be not higher than -60 °C. After the addition, the reaction solution was stirred for 2 h while the temperature was kept, and the reaction was then quenched with aqueous ammonium chloride solution. The resulting mixture was warmed to room temperature, and the mixture was added with 100 mL of water and then extracted twice with ethyl acetate. The combined organic phases were washed once with saturated sodium chloride, dried over anhydrous sodium sulfate and then concentrated to become dry. The resulting residue was slurried with ethyl acetate/petroleum ether to give the target product (1.6 g). ;

LC-MS: m/z 316 (M+H)⁺.

### Step 3: Preparation of 4-hydrazino-1-(methyl-d3)-1H-pyrazole

Di-*t*-butyl 1-(1-(methyl-*d*3)-1*H*-pyrazole-4-yl)hydrazino-1,2-dicarboxylate (1.6 g, 5.07 mmol) was added into HCl/dioxane (4 N, 40 mL), and the solution was stirred at room temperature for 16 h, then heated to 55 °C and stirred for another 4 h. The resulting reaction solution was concentrated under vacuum to become dry to give the target solid product (1.2 g). The product was directly used in the next reaction without purification.

LC-MS: m/z 116 (M+H)⁺.

### Example 2

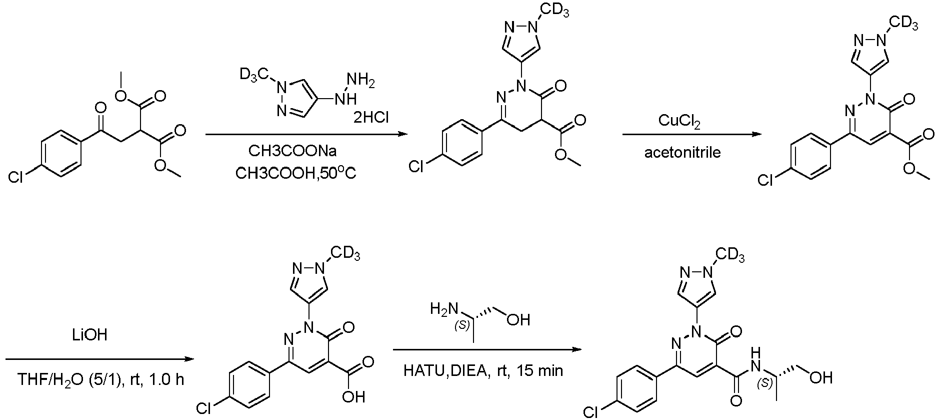

### Step 1: Preparation of Methyl 6-(4-chlorophenyl)-2-(1-(methyl-d3)-1H-pyrazole-4-yl)-3-oxo-2,3,4,5-tetrahydropyridazine-4-carboxylate

Dimethyl 2-(2-(4-chlorophenyl)-2-oxoethyl)malonate (809 mg, 2.84 mmol) was added into acetic acid (12 mL), and sodium acetate (746 mg, 9.09 mmol) and 4-hydrazino-1-(methyl-*d*3)-1*H-*pyrazole (641 mg, 3.41 mmol) were then added in sequence. After the addition, the reaction solution was stirred at room temperature for 1 h and then stirred at 50 °C overnight. The resulting reaction solution was concentrated, an excessive amount of sodium bicarbonate solution was added to regulate pH to 8, and extraction was performed with ethyl acetate (3 × 30 mL). The combined organic phases were washed once with saturated sodium chloride, dried over anhydrous sodium sulfate and then concentrated to become dry. The residue was separated by a preparative liquid phase to give the target product (357 mg, yield: 36%).

LC-MS: m/z 350 (M+H)⁺.

### Step 2: Preparation of Methyl 6-(4-chlorophenyl)-2-(1-(methyl-d3)-1H-pyrazole-4-yl)-3-oxo-2,3-dihydropyridazine-4-carboxylate

Methyl 6-(4-chlorophenyl)-2-(1-(methyl-*d*3)-1*H*-pyrazole-4-yl)-3-oxo-2,3,4,5-tetrahydropyridazine-4-carboxylate (357 mg, 1.02 mmol) was added into acetonitrile (16 mL), and anhydrous copper chloride (411 mg, 3.06 mmol) was added. After the addition, the reaction solution was warmed to 90°C and stirred for 2 h. The mixture was cooled to room temperature and concentrated under reduced pressure to remove the solvent. The residue was slurried with water to give the target product (230 mg, yield: 64.7%). The product was directly used in the next reaction without further purification.

LC-MS: m/z 348 (M+H)⁺.

### Step 3: Preparation of 6-(4-chlorophenyl)-2-(1-(methyl-d3)-1H-pyrazole-4-yl)-3-oxo-2,3-dihydropyridazine-4-carboxylate

Methyl 6-(4-chlorophenyl)-2-(1-(methyl-*d*3)-1*H*-pyrazole-4-yl)-3-oxo-2,3-dihydropyridazine-4-carboxylate (230.0 mg, 0.66 mmol) was dissolved in THF (3.0 mL), and an aqueous solution (0.6 mL) of LiOH·H₂O (83.0 mg, 1.99 mmol) was then added dropwise into the reaction system. The resulting reaction solution was stirred at room temperature for 1.0 h, insoluble material was then removed by filtration, and solid was washed with THF. The combined filtrates were concentrated, pH was then regulated to 3-4 with an aqueous solution of HCl (1 M), and extraction was performed with ethyl acetate (3 × 10 mL). The combined organic phases were washed with saturated brine, dried over anhydrous sodium sulfate and then concentrated under reduced pressure to give the target product (197.0 mg, yield: 89.4%). The product was directly used in the next reaction without further purification.

LC-MS: m/z 334 (M+H)⁺.

### Step 4: Preparation of (S)-6-(4-chlorophenyl)-N-(1-hydroxypropan-2-yl)-2-(1-(methyl-d3)-1H-pyrazole-4-yl)-3-oxo-2,3-dihydropyridazine-4-carboxamide

6-(4-chlorophenyl)-2-(1-(methyl-*d*3)-1*H*-pyrazole-4-yl)-3-oxo-2,3-dihydropyridazine-4-carboxylate (197.0 mg, 0.59 mmol) was dissolved in DMF (4.0 mL), and L-aminopropanol (97.5 mg, 1.3 mmol), DIPEA (0.3 mL, 1.95 mmol) and HATU (493.8 mg, 1.3 mmol) were added in sequence. The reaction solution was stirred at room temperature for 15 min, water (15 mL) was then added for quenching, and extraction was performed with ethyl acetate (3 × 10 mL). The combined organic phases were washed with saturated brine, dried over anhydrous sodium sulfate and then concentrated under reduced pressure. The resulting residue was separated by a liquid phase to give the target product (115.0 mg, yield: 50%).

LC-MS: m/z 391 (M+H)⁺. ¹H NMR (400 MHz, DMSO) δ 9.52 (d, *J =* 7.8 Hz, 1H), 8.56 (d, *J* = 9.5 Hz, 2H), 8.17 - 7.98 (m, 3H), 7.59 (d, *J=* 8.5 Hz, 2H), 4.96 (s, 1H), 4.06 (s, 1H), 3.48 (m, 2H), 1.19 (d, *J* = 6.6 Hz, 3H).

**According to the method of Example 2, the following compounds were synthesized from different starting materials:**

### Example 3: Preparation of 6-(4-chlorophenyl)-N-((trans)-2-hydroxycyclohexyl)-2-(1-(methyl-d3)-1H-pyrazole-4-yl)-3-oxo-2,3-dihydropyridazine-4-carboxamide

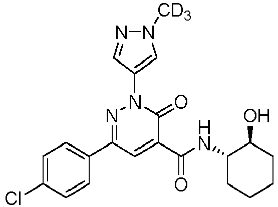

LC-MS: m/z 431 (M+H)⁺. ¹H NMR (400 MHz, DMSO) δ 9.51 (d, *J=* 7.7 Hz, 1H), 8.57 (d, *J* = 7.8 Hz, 2H), 8.19 - 8.01 (m, 3H), 7.60 (d, *J=* 8.6 Hz, 2H), 4.84 (d, *J=* 5.1 Hz, 1H), 3.74 - 3.58 (m, 1H), 3.38 (m, 1H), 2.10 - 1.92 (m, 1H), 1.88 (d, *J* = 8.7 Hz, 1H), 1.64 (m, 2H), 1.39 - 1.11 (m, 4H).

### Example 4: Preparation of 6-(4-chlorophenyl)-N-((trans)-3-hydroxytetrahydro-2H-pyran-4-yl)-2-(1-(methyl-d3)-1H-pyrazole-4-yl)-3-oxo-2,3-dihydropyridazine-4-carboxamide

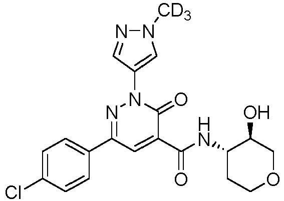

LC-MS: m/z 433 (M+H)⁺. ¹H NMR (400 MHz, DMSO) δ 9.55 (d, *J* = 7.3 Hz, 1H), 8.57 (d, *J* = 8.5 Hz, 2H), 8.22 - 7.96 (m, 3H), 7.60 (d, *J* = 8.4 Hz, 2H), 5.17 (d, *J* = 5.4 Hz, 1H), 3.95 - 3.69 (m, 3H), 3.52 - 3.34 (m, 2H), 3.11 (m, 1H), 2.03 (m, 1H), 1.51 (m, 1H).

### Example 5: Preparation of 6-(4-chlorophenyl)-N-((trans)-2-hydroxycyclopentyl)-2-(1-(methyl-d3)-1H-pyrazole-4-yl)-3-oxo-2,3-dihydropyridazine-4-carboxamide

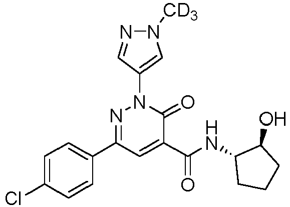

LC-MS: m/z 417 (M+H)⁺. ¹H NMR (400 MHz, DMSO) δ 9.44 (d, *J* = 7.4 Hz, 1H), 8.55 (d, *J* = 3.6 Hz, 2H), 8.14 - 7.97 (m, 3H), 7.60 (d, *J* = 8.6 Hz, 2H), 4.96 (d, *J* = 4.3 Hz, 1H), 4.12 - 3.85 (m, 2H), 2.07 (m, 1H), 1.91 - 1.80 (m, 1H), 1.78 - 1.60 (m, 2H), 1.60 - 1.34 (m, 2H).

### Example 6: Preparation of 6-(4-chlorophenyl)-N-((trans)-4-hydroxytetrahydrofuran-3-yl)-2-(1-(methyl-d3)-1H-pyrazole-4-yl)-3-oxo-2,3-dihydropyridazine-4-carboxamide

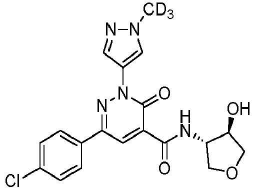

LC-MS: m/z 419 (M+H)⁺. ¹H NMR (400 MHz, DMSO) δ 9.56 (d, *J* = 7.4 Hz, 1H), 8.56 (s, 2H), 8.10 (d, *J* = 9.2 Hz, 3H), 7.60 (d, *J* = 8.6 Hz, 2H), 5.50 (d, *J* = 3.8 Hz, 1H), 4.26 (dd, *J* = 6.9, 5.0 Hz, 1H), 4.20 (s, 1H), 3.99 (dd, *J* = 9.2, 4.8 Hz, 1H), 3.93 (dd, *J* = 9.6, 4.6 Hz, 1H), 3.68 (dd, *J* = 9.2, 1.8 Hz, 1H), 3.56 (dd, *J* = 9.6, 1.7 Hz, 1H).

### Example 7: Preparation of (S)-6-(4-chlorophenyl)-N-(1-hydroxypropan-2-yl-1,1-d2)-2-(1-methyl-1H-pyrazole-4-yl)-3-oxo-2,3-dihydropyridazine-4-carboxamide

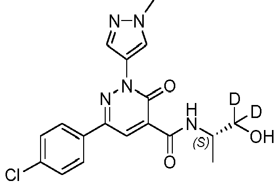

LC-MS: m/z 390 (M+H)⁺. ¹HNMR(400M, CDCl₃) □ 9.81 (d, *J* = 8.0 Hz,1H), 8.68 (s,1H), 8.35 (s,1H), 8.12 (s, 1H), 7.88 (d, *J* = 8.0 Hz, 2H), 7.50 (d, *J* = 8.0 Hz, 2H), 4.29 (m,1H), 3.98 (s, 3H), 1.34 (d, *J* = 4 Hz, 3H).

### Example 8: Preparation of (S)-6-(4-chlorophenyl)-N-(1-hydroxypropan-2-yl-1,1-d2)-2-(1-(methyl-d3)-1H-pyrazole-4-yl)-3-oxo-2,3-dihydropyridazine-4-carboxamide

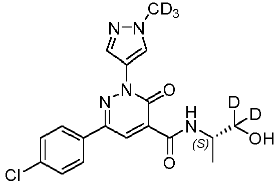

LC-MS: m/z 393 (M+H)⁺. ¹H NMR (400 MHz, DMSO) δ 9.52 (d, *J* = 7.8 Hz, 1H), 8.57 (d, *J* = 9.1 Hz, 2H), 8.16 - 8.03 (m, 3H), 7.60 (d, *J* = 8.6 Hz, 2H), 4.91 (s, 1H), 4.04 (p, *J* = 6.7 Hz, 1H), 1.19 (d, *J* = 6.7 Hz, 3H).

### Example 9: Preparation of (S)-6-(4-chlorophenyl)-N-(1-hydroxypropan-2-yl)-2-(1-methyl-1H-pyrazole-4-yl)-3-oxo-2,3-dihydropyridazine-5-d-4-carboxamide

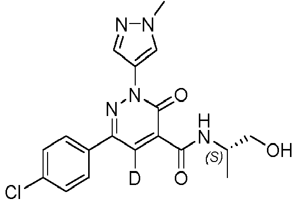

LC-MS: m/z 389 (M+H)⁺. ¹H NMR (400 MHz, DMSO) δ 9.52 (d, *J* = 7.9 Hz, 1H), 8.58 (s, 1H), 8.14 - 8.04 (m, 3H), 7.60 (d, *J* = 8.5 Hz, 2H), 4.95 (t, *J* = 5.2 Hz, 1H), 4.06 (m, 1H), 3.93 (s, 3H), 3.48 (s, 2H), 1.19 (d, *J* = 6.7 Hz, 3H).

### Example 10: Preparation of (S)-6-(4-chlorophenyl)-N-(1-hydroxypropan-2-yl-1,1-d2)-2-(1-methyl-1H-pyrazole-4-yl)-3-oxo-2,3-dihydropyridazine-5-d-4-carboxamide

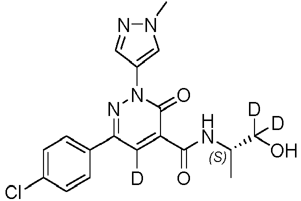

LC-MS: m/z 391 (M+H)⁺.

### Example 11: Preparation of (S)-6-(4-chlorophenyl)-N-(1-hydroxy-3-methylbutan-2-yl)-2-(1-(methyl-d3)-1H-pyrazole-4-yl)-3-oxo-2,3-dihydropyridazine-4-carboxamide

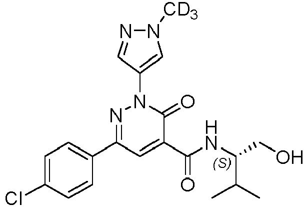

LC-MS: m/z 419 (M+H)⁺. ¹H NMR (400 MHz, DMSO) δ 9.53 (d, *J* = 9.2 Hz, 1H), 8.59 (d, *J* = 9.1 Hz, 2H), 8.19 - 8.02 (m, 3H), 7.61 (d, *J* = 8.6 Hz, 2H), 4.83 (t, *J* = 5.3 Hz, 1H), 3.87 (m, 1H), 3.58 (m, 1H), 3.48 (m, 1H), 2.00 (m, 1H), 1.01 - 0.88 (m, 6H).

### Example 12: Preparation of (S)-6-(4-chlorophenyl)-N-(1-cyclopropyl-2-hydroxyethyl)-2-(1-(methyl-d3)-1H-pyrazole-4-yl)-3-oxo-2,3-dihydropyridazine-4-carboxamide

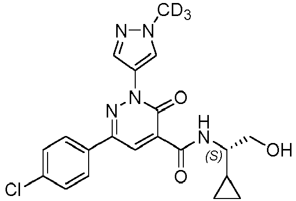

LC-MS: m/z 417(M+H)⁺. ¹H NMR (400 MHz, DMSO) δ 9.66 (d, *J* = 8.6 Hz, 1H), 8.58 (d, *J* = 6.7 Hz, 2H), 8.22 - 8.00 (m, 3H), 7.60 (d, *J* = 8.6 Hz, 2H), 4.94 (t, *J* = 5.3 Hz, 1H), 3.69 - 3.53 (m, 2H), 3.46 (m, 1H), 1.17 - 1.03 (m, 1H), 0.53 - 0.23 (m, 4H).

### Example 13: Preparation of (S)-6-(4-chlorophenyl)-N-(1-hydroxypropan-2-yl-1,1,2,3,3,3-d6)-2-(1-methyl-1H-pyrazole-4-yl)-3-oxo-2,3-dihydropyridazine-4-carboxamide

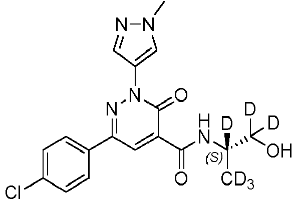

LC-MS: m/z 394 (M+H)⁺.

### Example 14: Preparation of (S)-6-(4-chlorophenyl)-N-(1-hydroxypropan-2-yl-1,1,2,3,3,3-d6)-2-(1-(methyl-d3)-1H-pyrazole-4-yl)-3-oxo-2,3-dihydropyridazine-4-carboxamide

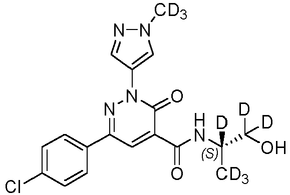

LC-MS: m/z 397 (M+H)⁺.

### Example 15: Preparation of (S)-6-(4-chlorophenyl-2,3,5,6-d4)-N-(1-hydroxypropan-2-yl)-2-(1-methyl-1H-pyrazole-4-yl)-3-formyl-2,3-dihydropyridazine-4-carboxamide

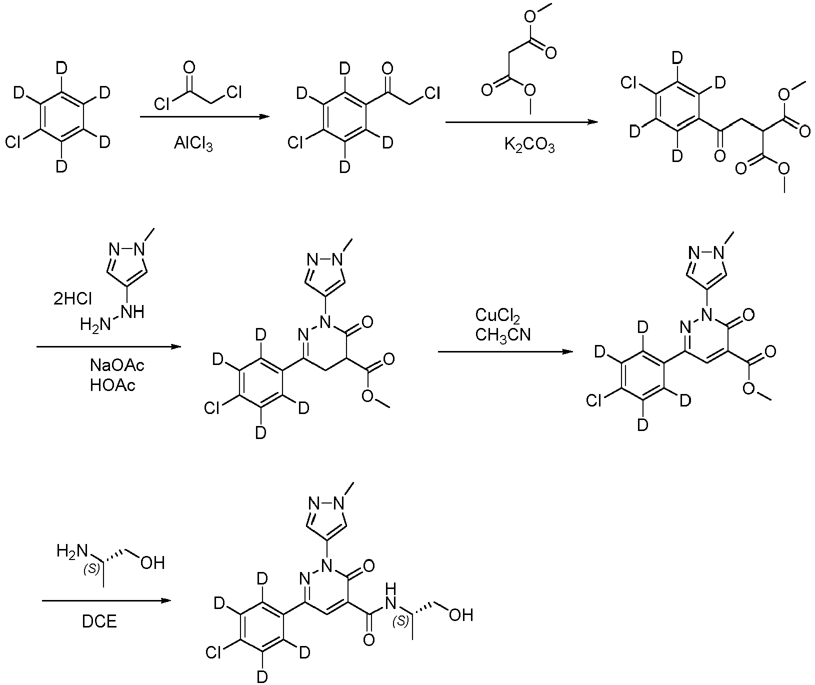

### Step 1: Preparation of 2-chloro-1-(4-chlorophenyl-2,3,5,6-d4)ethyl-1-one

The compounds 1-chlorobenzene-2,3,4,5,6-*d*5 (5 g, 42.5 mmol) and dichloromethane (35 mL) were added in sequence into a round-bottom flask. The reaction solution was cooled to 0 °C in an ice bath, the compound chloroacetyl chloride (5.8 g, 51 mmol) was then added, and finally aluminum trichloride was added (9 g, 68 mmol). The resulting reaction solution reacted at 0 °C for 2 h, and was then added into ice water for quenching and extracted with dichloromethane. The combined organic phases were dried over anhydrous magnesium sulfate, filtered and concentrated to give the target compound (7.4 g, yield: 90.7%). The product was directly used in the next reaction without purification.

LC-MS: m/z 191 (M-H)⁻.

### Step 2: Preparation of Dimethyl 2-(2-(4-chlorophenyl-2,3,5,6-d4)-2-formylmethyl)malonate

The compounds 2-chloro-1-(4-chlorophenyl-2,3,5,6-*d*4)ethyl-1-one (7.4 g, 38 mmol), dimethyl malonate (25 g, 191 mmol), potassium carbonate (26 g, 191 mmol) and acetone (150 mL) were added in sequence into a round-bottom flask. The reaction solution was reacted at room temperature for 20 h and was then concentrated. The residue was extracted with ethyl acetate after water was added. The combined organic phases were dried over anhydrous magnesium sulfate, filtered and concentrated. The resulting crude product was separated by silica gel column chromatography (ethyl acetate/petroleum ether = 1/5) to give the target compound (9.3 g, yield: 84.7%).

LC-MS: m/z 289 (M+H)⁺.

### Step 3: Preparation of Methyl 6-(4-chlorophenyl-2,3,5,6-d4)-2-(1-methyl-1H-pyrazole-4-yl)-3-formyl-2,3,4,5-tetrahydropyridazine-4-carboxylate

The compounds dimethyl 2-(2-(4-chlorophenyl-2,3,5,6-*d*4)-2-formylmethyl)malonate (130 mg, 0.45 mmol), 4-hydrazino-1-methyl-1*H*-pyrazole dihydrochloride (100 mg, 0.54 mmol), sodium acetate (118 mg, 1.44 mmol) and acetic acid (3 mL) were added in sequence into a round-bottom flask. The reaction solution was reacted at room temperature for 1 h, and was then warmed to 50 °C to react for 20 h. The resulting reaction solution was concentrated and added with water, pH was then regulated to 8, and extraction was performed with ethyl acetate. The combined organic phases were dried over anhydrous magnesium sulfate, filtered and concentrated. The resulting crude product was separated by silica gel column (ethyl acetate/petroleum ether = 1/3) to give the target compound (47 mg, yield: 29.8%).

LC-MS: m/z 351 (M+H)⁺.

### Step 4: Preparation of Methyl 6-(4-chlorophenyl-2,3,5,6-d4)-2-(1-methyl-1H-pyrazole-4-yl)-3-formyl-2,3-dihydropyridazine-4-carboxylate

The compounds methyl 6-(4-chlorophenyl-2,3,5,6-*d*4)-2-(1-methyl-1*H*-pyrazole-4-yl)-3-formyl-2,3,4,5-tetrahydropyridazine-4-carboxylate (47 mg, 0.13 mmol), copper chloride (54 mg, 0.40 mmol) and acetonitrile (4 mL) were added in sequence into a round-bottom flask. The reaction solution was warmed to 90 °C to react for 4 h, concentrated and then added with water. The resulting mixture was stirred and then filtered, and the filter cake was washed with water and dried to give the target compound (50 mg, quantitative yield).

LC-MS: m/z 349 (M+H)⁺.

### Step 5: Preparation of (S)-6-(4-chlorophenyl-2,3,5,6-d4)-N-(1-hydroxypropan-2-yl)-2-(1-methyl-1H-pyrazole-4-yl)-3-formyl-2,3-dihydropyridazine-4-carboxamide

The compounds methyl 6-(4-chlorophenyl-2,3,5,6-*d*4)-2-(1-methyl-1*H*-pyrazole-4-yl)-3-formyl-2,3-dihydropyridazine-4-carboxylate (50 mg, 0.14 mmol), L-aminopropanol (16 mg, 0.22 mmol) and dichloroethane (1 mL) were added in sequence into a round-bottom flask. The reaction solution was reacted at 50 °C for 3 h and was then concentrated, and the residue was separated by a preparative liquid phase to give the target compound (27 mg, yield: 49.2%).

LC-MS: m/z 392 (M+H)⁺. ¹H NMR (400 MHz, DMSO-d6) δ 9.52 (d, *J* = 8.0Hz, 1H), 8.58 (s, 1H), 8.56 (s, 1H), 8.12 (s, 1H), 4.95 (t, *J* = 5.2Hz, 1H), 4.10-4.01(m, 1H), 3.93 (s, 3H),3.49-3.46 (m, 2H),1.19 (d, *J* = 6.8Hz, 3H).

**According to the method of Example 15, the following compounds were synthesized from different starting materials:**

### Example 16: Preparation of (S)-6-(4-chlorophenyl-2,3,5,6-d4)-N-(1-hydroxypropan-2-yl)-2-(1-(methyl-d3)-1H-pyrazole-4-yl)-3-oxo-2,3-dihydropyridazine-4-carboxamide

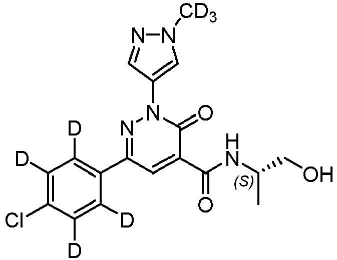

LC-MS: m/z 395 (M+H)⁺.

### Example 17: Preparation of (S)-6-(4-chlorophenyl-2,3,5,6-d4)-N-(1-hydroxypropan-2-yl-1,1-d2)-2-(1-(methyl-d3)-1H-pyrazole-4-yl)-3-oxo-2,3-dihydropyridazine-4-carboxamide

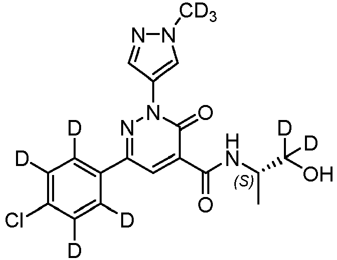

LC-MS: m/z 397 (M+H)⁺.

### Example 18: Biological Test and Evaluation

The present invention is further described and explained below with reference to biological test examples, but these examples are not intended to limit the scope of the present invention.

### Antagonistic Study of AhR β-lactamase

### Preparation of Reagents

(1) Breeding medium: Opti-MEM, 5% FBS, 1 uM sodium Pyruvate, 0.1 mM NEAA, stored at 4°C.
(2) Experimental medium: Opti-MEM, 0.1% BSA, 1 uM sodium Pyruvate, 0.1 mM NEAA, stored at 4°C.

### Procedure:

### Day 1:

### Coating a test plate (the day before the test)

1) 25 mg of poly-L-lysine was dissolved in 500 mL of DPBS;
2) 20 uL was added per well, and incubation was carried out under the conditions of 37 °C and 5% CO₂ for 1-2 h;
3) the solution was completely poured, and washing was carried out once with 50 uL of MEM.

### Seeding cells on plates

1) cells of the CYP1A1-bla LS-180 cell line were taken out of the incubator;
2) the medium was removed, and 2 mL of TrypLE was added per flask;
3) the flask was allowed to stand at 37 °C for 5-8 min to separate the cells;
4) 10 mL of seeding medium was added per flask;
5) the cell mixture was transferred into a 50 ml sterile centrifuge tube and uniformly stirred;
6) the cells were counted and spread onto cell plates;
7) 15K cells/well: 15K cells were put into each well with 30 uL of medium;
8) the cells were seeded into the wells by using 30 uL of breeding medium, each well adopting the Combi standard mode of a biomembrane poly-D-lysine 384-well TC-treated microplate;
9) incubation was carried out under the conditions of 37 °C and 5% CO₂ overnight.

### Day 2:

### Administering the compounds

a) preparation of reference compound
   20 doses of reference CH-223191 was prepared in LDV plates with DMSO. the maximum working solution of CH-223191 was a 10 mM DMSO solution. 2-fold serial dilution was carried out with Bravo.
b) Max and Min Wells
   Max Wells: 10 mM CH-223191 in DMSO standard solution was in the LDV plates;
   Min Wells: a pure DMSO solution was in the LDV plates.
c) 80 nLs were transported from the LDV plates into composite plates (PE6008590) using ECHO according to plate mapping.
d) adopting the Combi Stand mode, 20 uL of detection medium was added into each well and centrifuged at 2000 rpm for 2 min for uniform mixing.
e) starvation of cells
   29 uL of medium was transferred into the wells of the cell plates by using Bravo;
   adopting the Combi Stand mode, 20 uL of warm test solution was added into each well and centrifuged at 300 rpm for 1 min;
   incubation was carried out under the conditions of 37 °C and 5% CO₂ for 1 h.
f) addition of compound
   10 uL of dilution or DMSO (4-fold working solution) was transferred from the composite plates (PE6008590) into the cell plates by using Bravo.
   incubation was carried out under the conditions of 37 °C and 5% CO₂ for 1 h.
g) addition of receptor agonist
   10 uL of 4X ITE working solution (2 nM) was added into all the wells of the cell plates by using Bravo. the total detection system was 40 uL/Well.
   incubation was carried out under the conditions of 37 °C and 5% CO₂ for 4 h;
   the plates were allowed to stand at room temperature for 10 min before the loading reagents were added.
h) final assay conditions final well: 15 k/well
   Min: 0.1% DMSO solution
   Max: 10 uM CH-223191
   CH-223191 CRC: diluted 2-fold to 10 uM, 20 points final ITE concentration: 0.5 nM

### Day 2: Adding assay buffer and reading the plates

### A. Loading substrates and reading the plates

a) each reagent was stored and prepared according to the instructions of the kit.
b) the solution A and the solution D were taken out of a -20 °C refrigerator. after complete thawing, a mixed solution was prepared as follows:
c) the solution A was added into the solution B and vortexed well;
d) the solution C was added into the mixture of A and B and vortexed well;
e) the solution D was added into the solution of A, B, and C and vortexed well;
f) 9.5 uL of final solution was added into each plate by adopting the Combi mini-mode;
g) incubation was carried out at room temperature in the dark for at least 1 h, and the plates were read. the signal could operate for at least 3 h.

### B. Reading the plates by adopting Envision

### Settings of Envision Reader

| | Wavelength |
|---|---|
| Excitation Filter | 400 |
| Emission Filter 1 | 460 |
| Emission Filter 2 | 535 |
| Dichroic Mirror | Beta lactamase D425/490 |

Antagonistic activity of some of the compounds of the examples disclosed herein against AhR are shown in Table 1.

**Table 1: Inhibitory Activity of Compounds of Examples of the Present Invention**

| **IC₅₀** | (nM) |
|---|---|
| BAY-2416964 | <100 |
| **Example 2** | <50 |
| **Example 7** | <50 |
| **Example 8** | <50 |

As can be seen from Table 1, the compounds of the examples disclosed herein show good antagonistic activity against AhR.

### Pharmacokinetic Test and Evaluation in Rats

After male SD rats weighing about 220 g were fasted overnight, the rats were gavaged with 2 mg/kg of a solution of the compound of the present invention [CMC/TW80 as a carrier]. Blood was collected 0.5, 1.0, 2.0, 4.0, 6.0, 8.0, 12 and 24 h after the administration of the compound of the present invention, respectively, and the concentration of the compound of the present invention in plasma was determined by LC/MS/MS.

### Reference compound: BAY-2416964

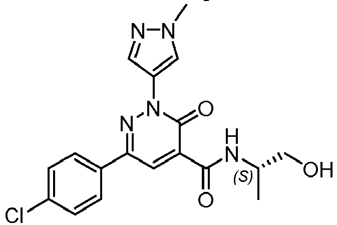

**Table 1: Summary of Pharmacokinetic Parameters (n = 4, mean)**

| Parameter (s) | Unit | BAY-2416964 | **Example 2** | **Example 7** | **Example 8** |
|---|---|---|---|---|---|
| Administration dose | | 2 mg/kg | 2 mg/kg | 2 mg/kg | 2 mg/kg |
| AUC₀₋ₜ | ng.h/mL | 624 | 990 | 736 | 1394 |
| Cmax | ng/mL | 99.5 | 146 | 96.6 | 230 |

Relative to the control compound BAY-2416964, the compounds of the **examples** obtained by the present invention show better metabolic properties in the rat body, and have higher plasma exposures AUC and Cmax, thus having better pharmaceutical efficacy.

### Pharmacokinetic Test and Evaluation in Mice

After male ICR mice weighing about 20-30 g were fasted overnight, the mice were gavaged with 2 mg/kg of a solution of the compound of the present invention [CMC/TW80 as a carrier]. Blood was collected 0.5, 1.0, 2.0, 4.0, 6.0, 8.0, 12 and 24 h after the administration of the compound of the present invention, respectively, and the concentration of the compound of the present invention in plasma was determined by LC/MS/MS.

**Table 2: Summary of Pharmacokinetic Parameters (n = 4, mean)**

| Parameter (s) | Unit | BAY-2416964 | **Example 2** | **Example 7** | **Example 8** |
|---|---|---|---|---|---|
| Administration dose | | 2 mg/kg | 2 mg/kg | 2 mg/kg | 2 mg/kg |
| AUC₀₋ₜ | ng.h/mL | 1490 | 850 | 2456 | 2617 |
| Cmax | ng/mL | 348 | 241 | 351 | 351 |

Relative to the control compound BAY-2416964, the compounds of the **examples** obtained by the present invention show better metabolic properties in the mouse body, and have higher plasma exposure AUC, thus having better pharmaceutical efficacy.

### Pharmacokinetic Test and Evaluation in Beagle Dogs

After male beagle dogs weighing about 6-7 Kg were fasted overnight, the beagle dogs were gavaged with 2 mg/kg of a solution of the compound of the present invention [CMC/TW80 as a carrier]. Blood was collected 0.5, 1.0, 2.0, 4.0, 6.0, 8.0, 12 and 24 h after the administration of the compound of the present invention, respectively, and the concentration of the compound of the present invention in plasma was determined by LC/MS/MS.

**Table 3: Summary of Pharmacokinetic Parameters (n = 4, mean)**

| Parameter (s) | Unit | BAY-2416964 | **Example 8** |
|---|---|---|---|
| Administration dose | | 2 mg/kg | 2 mg/kg |
| AUC₀₋ₜ | ng.h/mL | 995 | 1875 |
| Cmax | ng/mL | 211 | 294 |

Relative to the control compound BAY-2416964, the compounds of the **examples** obtained by the present invention show better metabolic properties in the dog body, and have higher plasma exposures AUC and Cmax, thus having better pharmaceutical efficacy.

### Pharmacodynamic Test and Evaluation of Anti-tumor Activity

### 1. Subcutaneous Transplantation Cancer Model CT26.WT

5×10⁵/100 uL of mouse colon cancer cells CT26.WT were subcutaneously inoculated into the right back of nude mice. The mice were monitored daily for health condition, and the measurement was started when tumors grew to be palpable. The tumor volume was calculated by adopting the following formula: 0.5×L×W², wherein L and W represent tumor length and width, respectively. When the tumor grew to about 50 mm³, the mice were randomly grouped. The mice were gavaged daily with a solution of the compound at a corresponding dose (30 mg/Kg) [CMC/TW80 as a carrier], and at the same time, the general state of the mice was monitored. After the administration was started, the mice were weighed twice in the first week, and the tumor volume was measured twice; from the second week, the mice were weighed three times a week, and the tumor volume was measured three times. The test results are shown in Table 4 and FIG. 1.

**Table 4: Pharmacodynamic Test and Evaluation of Anti-tumor Activity**

| **Group** | **TV (mm³) (Tumor Volume) Day 0** | **TV (mm³) (Tumor Volume) Day 17** | **%TGI (Tumor Growth Inhibition)** |
|---|---|---|---|
| Blank control group | 55.55 | 2200.23 | / |
| Example 8 | 55.68 | 1117.93 | 50.5% |
| BAY-2416964 | 55.81 | 1670.91 | 24.7% |

The results show that the compounds of the examples obtained by the present invention have better anti-tumor efficacy relative to the control compoundBAY-2416964.

All documents mentioned in the present invention are incorporated as references, just as each document is individually cited as a reference. In addition, it should be understood that various modifications or changes may be made by those skilled in the art after reading the above teachings of the present invention, and these equivalent forms also fall within the scope defined by the claims appended hereto.

## Claims

1. A deuterated 3-oxo-2,3-dihydropyridazine-4-carboxamide compound having a structure of general formula (I), or a stereoisomer, a tautomer, a crystalline form, a pharmaceutically acceptable salt, a hydrate, a solvate, or a prodrug thereof: 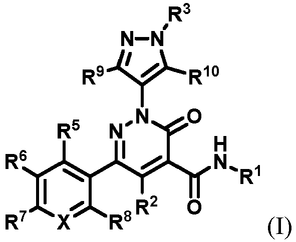 in the formula:
R¹ is selected from the group consisting of the following substituted groups: C₂-C₆ alkyl, C₃-C₁₀ cycloalkyl, and 4- to 10-membered heterocyclyl; wherein the above C₂-C₆ alkyl, C₃-C₁₀ cycloalkyl or 4- to 10-membered heterocyclyl is substituted with at least one hydroxyl group;
R² is selected from the group consisting of hydrogen and deuterium;
R³ is selected from the group consisting of the following substituted or unsubstituted groups: C₁-C₃ alkyl, C₃-C₆ cycloalkyl, and 4- to 6-membered heterocyclyl;
X is selected from the group consisting of N and CR⁴;
R⁴, R⁵, R⁶, R⁸, R⁹, or R¹⁰ is selected from the group consisting of hydrogen and deuterium;
R⁷ is selected from the group consisting of Cl, CF₃, CHF₂, OCF₃, OCHF₂, and N(Me)₂;
wherein the above substitution refers to a substitution by one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, nitro, hydroxy, cyano, ester, amino, amido, sulfonamido, and ureido;
the limiting condition is that at least one of R¹, R², R³, R⁴, R⁵, R⁶, R⁷, R⁸, R⁹, and R¹⁰ is deuterium or substituted with a deuterium atom.

2. The deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to claim 1, wherein the compound has a structure of general formula (II): 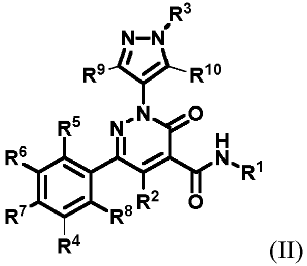 R¹, R², R³, R⁴, R⁵, R⁶, R⁷, R⁸, R⁹, and R¹⁰ are as defined in claim 1.

3. The deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to claim 1, wherein the compound has a structure of general formula (III): 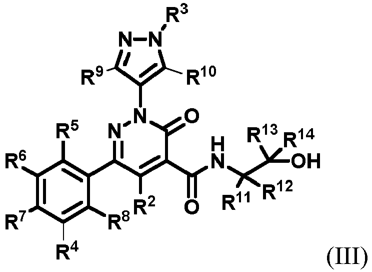 in the formula, R¹¹, R¹², R¹³, and R¹⁴ are each independently selected from the group consisting of the following substituted or unsubstituted groups: H, deuterium, cyano, C₁-C₆ alkyl, C₃-C₆ cycloalkyl, and 4- to 6-membered heterocyclyl; and R², R³, R⁴, R⁵, R⁶, R⁷, R⁸, R⁹, and R¹⁰ are as defined in claim 1.

4. The deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to claim 1, wherein the compound has a structure of formula IV: 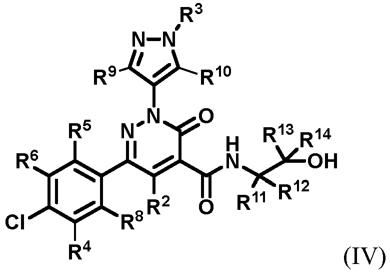 in the formula, R¹¹, R¹², R¹³, and R¹⁴ are each independently selected from the group consisting of the following substituted or unsubstituted groups: H, deuterium, cyano, C₁-C₆ alkyl, C₃-C₆ cycloalkyl, and 4- to 6-membered heterocyclyl; and R², R³, R⁴, R⁵, R⁶, R⁸, R⁹, and R¹⁰ are as defined in claims 1 and 3.

5. The deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to claim 1, wherein R³ is selected from the group consisting of: CH₃ and CD₃.

6. The deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to claim 1, wherein R¹³ and R¹⁴ are each independently deuterium.

7. The deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to claim 1, wherein the compound is selected from the group consisting of:

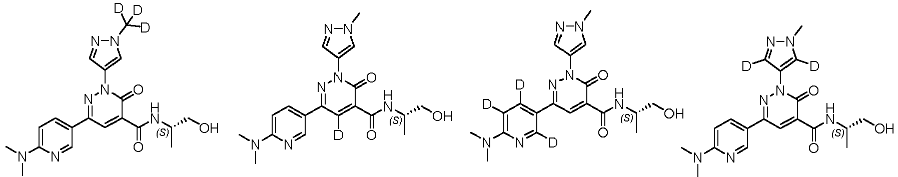

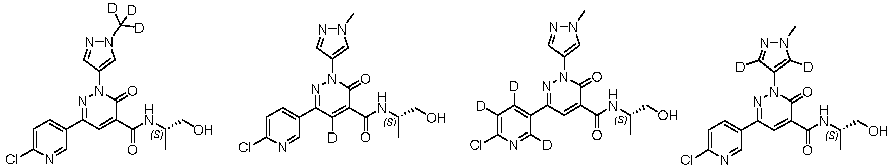

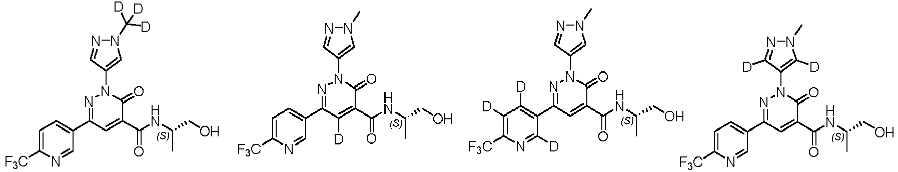

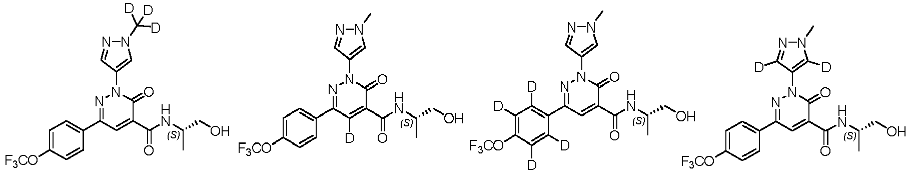

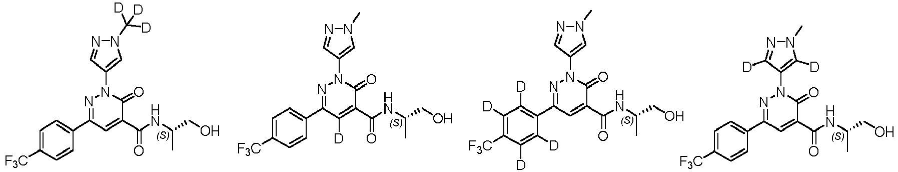

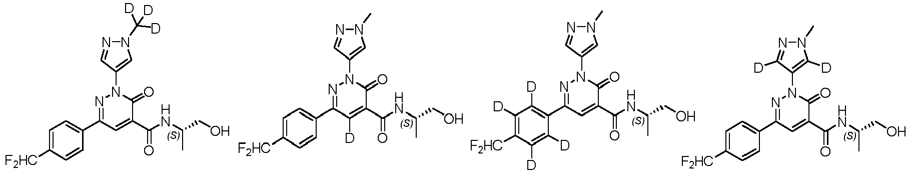

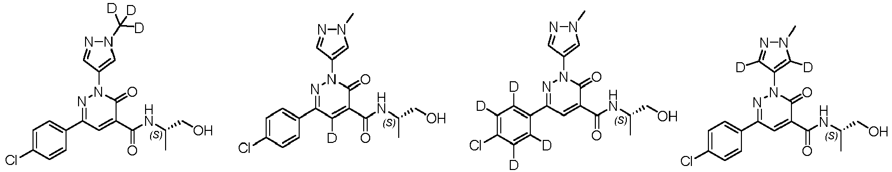

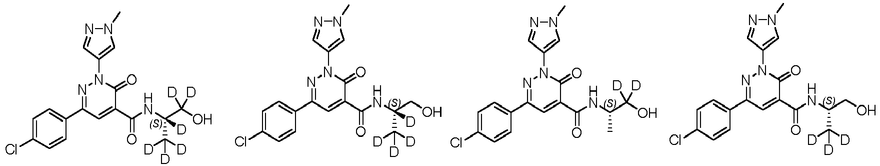

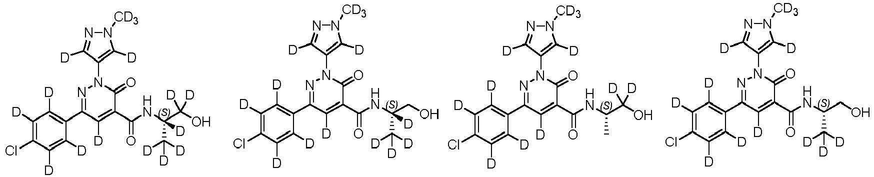

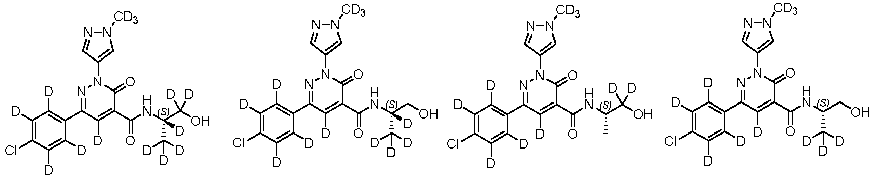

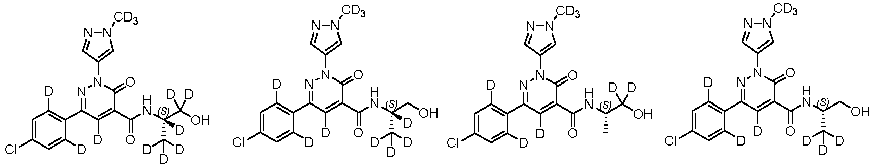

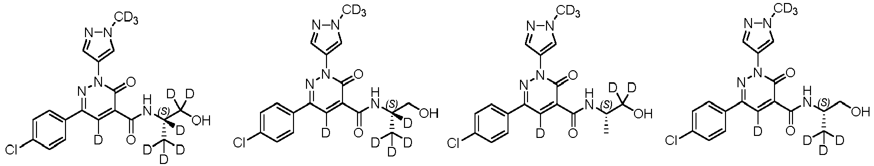

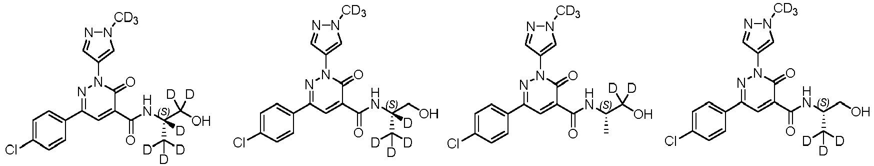

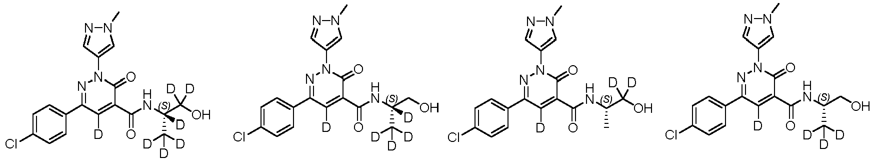

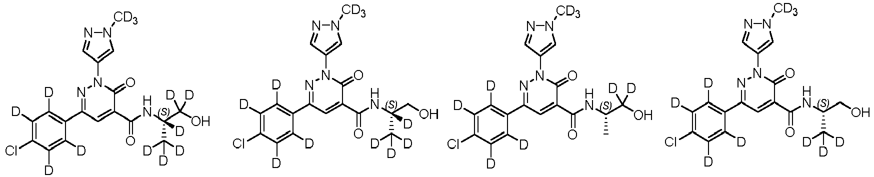

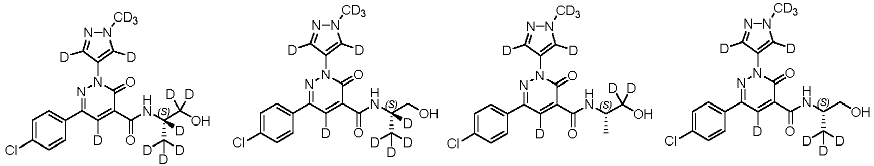

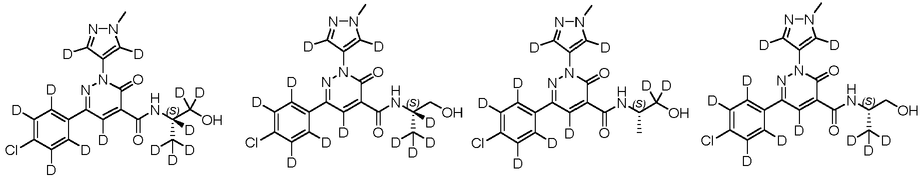

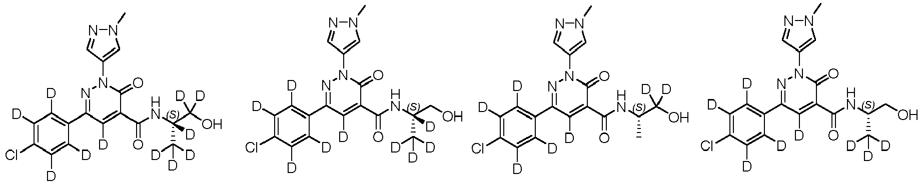

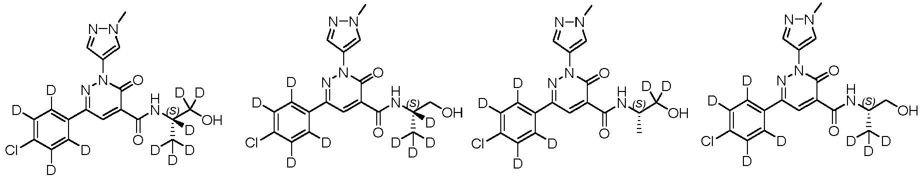

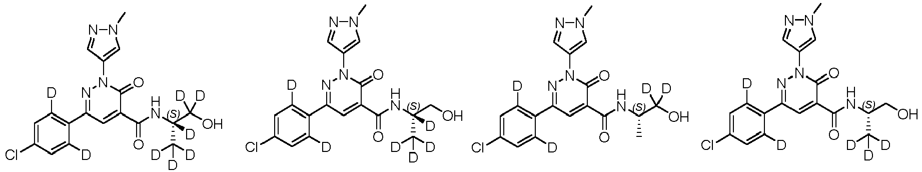

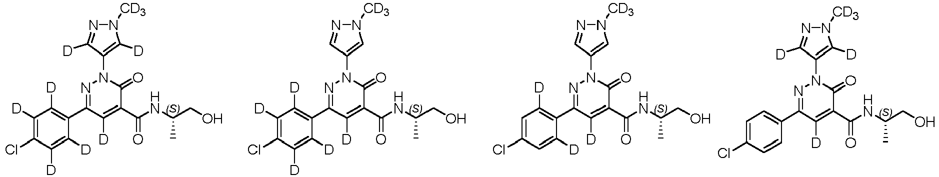

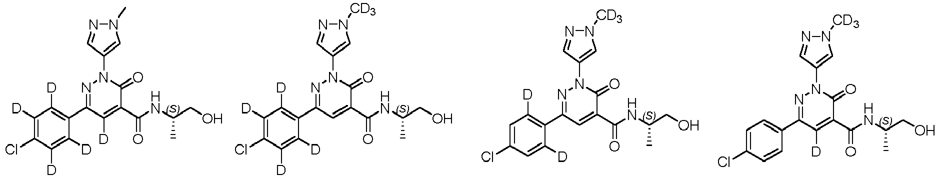

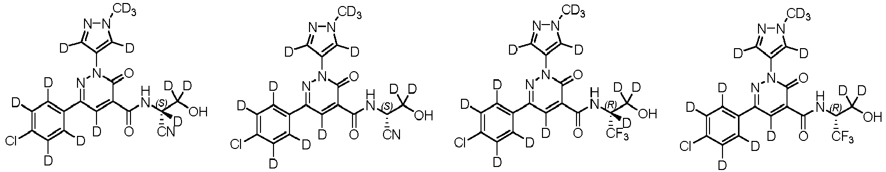

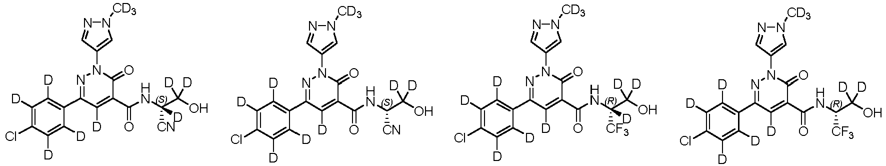

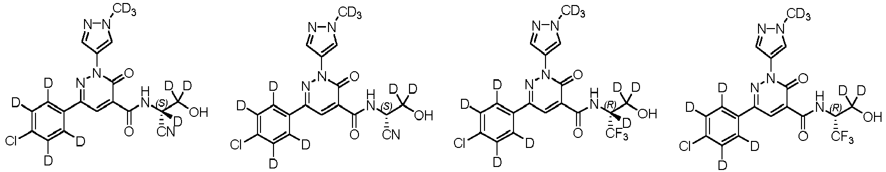

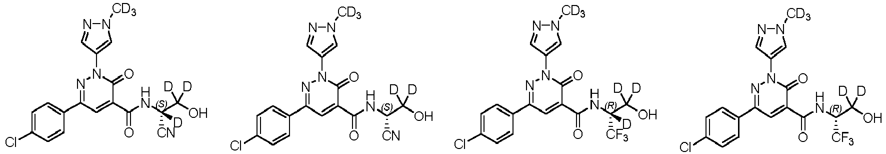

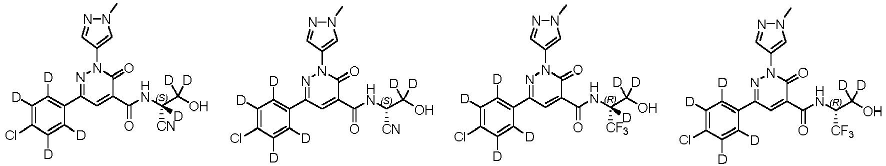

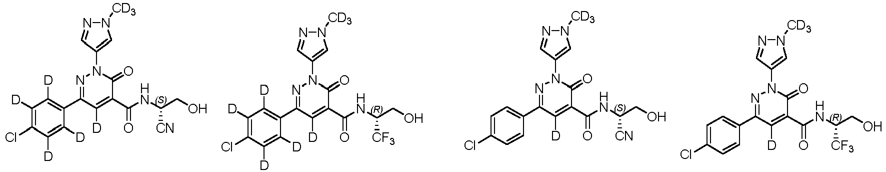

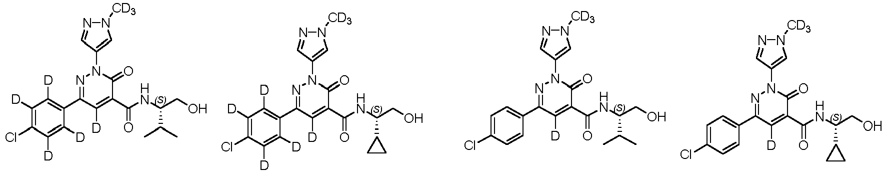

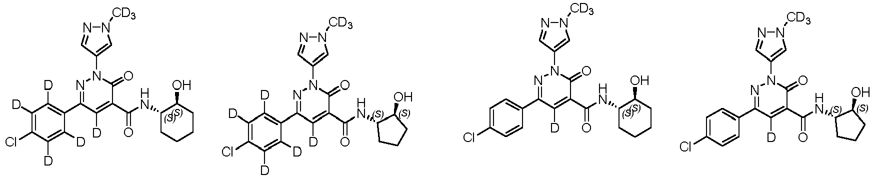

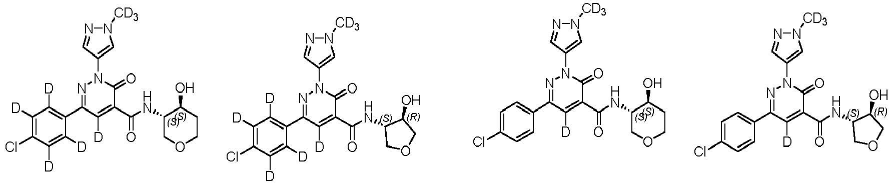

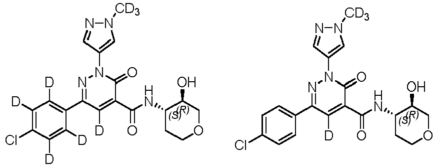

8. A method for preparing a deuterated 3-oxo-2,3-dihydropyridazine-4-carboxamide compound having a structure of general formula (I), or a stereoisomer, a tautomer, a crystalline form, a pharmaceutically acceptable salt, a hydrate, a solvate, or a prodrug thereof, comprising the steps of: 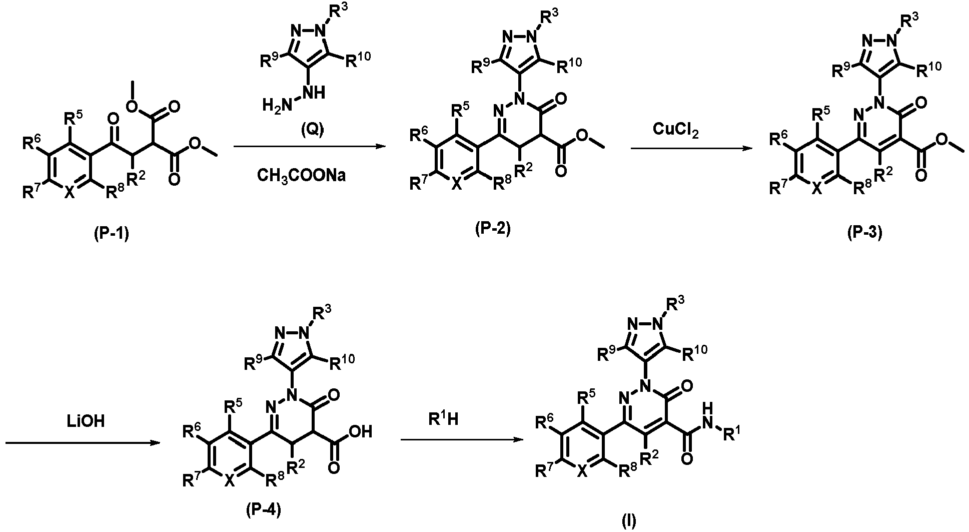
(i) a compound of formula (P-1) reacting with a compound of formula (Q) in the presence of a first base (e.g., sodium acetate) to give a compound of formula (P-2);
(ii) the compound of formula (P-2) carrying out dehydrogenation reaction in the presence of a copper salt (e.g., CuCh) to give a compound of formula (P-3);
(iii) hydrolyzing the compound of formula (P-3) in the presence of a second base (e.g., LiOH) to give a compound of formula (P-4);
(iv) the compound of formula (P-4) reacting with an amine (R¹H) to give the compound of formula (I);
in the formula,
R¹, R², R³, R⁵, R⁶, R⁷, R⁸, R⁹, R¹⁰, and X are as defined in claim 1.

9. A pharmaceutical composition, comprising: i) one or more of the compound having the structure of general formula (I), or the stereoisomer, the tautomer, the crystalline form, the pharmaceutically acceptable salt, the hydrate, the solvate, or the prodrug thereof according to any one of claims 1 to 5; and ii) a pharmaceutically acceptable carrier.

10. Use of the deuterated 3-oxo-2,3-dihydropyridazine-4-carboxamide compound having the structure of general formula (I), or the stereoisomer, the tautomer, the crystalline form, the pharmaceutically acceptable salt, the hydrate, the solvate, or the prodrug thereof according to any one of claims 1 to 7, or the pharmaceutical composition according to claim 9 in the preparation of a pharmaceutical composition for preventing and/or treating an AhR-mediated disease.
